# EUROPEAN PATENT SPECIFICATION

(11) **EP 2 344 043 B1**
(45) Date of publication and mention of the grant of the patent: **07.05.2014**
(21) Application number: 08786802.2
(22) Date of filing: 01.08.2008
(51) Int. Cl.: A61B 8/00, G01S 7/52, G01S 7/521, H05K 5/00, G06F 1/16

(54) **PORTABLE ULTRASOUND SYSTEM**
TRAGBARES ULTRASCHALLSYSTEM
SYSTEME ULTRASONORE PORTABLE

(43) Date of publication of application: 20.07.2011
(60) Divisional application: 14162188.8
(73) Proprietor: Esaote Europe B.V., 6227 Aj Maastricht (NL)
(72) Inventor: WILLEMS, John, NL-6227 Aj Maastricht (NL); GEIJSEN, Joop, NL-6227 Aj Maastricht (NL)
(74) Representative: Karaghiosoff, Giorgio Alessandro
(86) International application number: PCT/EP2008/060187
(87) International publication number: WO 2010/012314

(56) References cited:
- EP-A- 0 254 889
- EP-A- 0 965 785
- EP-A1- 1 925 257
- WO-A1-2006/040697
- DE-U1- 20 209 113
- US-A- 5 293 300
- US-A- 5 590 658
- US-A- 5 609 485
- US-A- 5 721 668
- US-A- 6 115 883
- US-A1- 2002 158 601
- US-A1- 2004 226 973
- US-A1- 2006 034 045
- US-A1- 2007 276 250
- US-A1- 2008 119 731
- US-A1- 2008 139 934
- US-A1- 2008 161 690
- US-B1- 6 301 101
- US-B1- 6 491 630
- "HANDLE USED AS BATTERY COMPARTMENT" IBM TECHNICAL DISCLOSURE BULLETIN, IBM CORP. NEW YORK, US, vol. 27, no. 11, 1 April 1985 (1985-04-01) , XP002017931 ISSN: 0018-8689
- EIDHEIM ET AL: "Real-time analysis of ultrasound images using GPU" INTERNATIONAL CONGRESS SERIES, EXCERPTA MEDICA, AMSTERDAM, vol. 1281, 1 May 2005 (2005-05-01), pages 284-289, XP005081682 ISSN: 0531-5131

## Description

The invention relates to a portable ultrasonic according to the preamble of claim 1.

Ultrasonic systems or devices of the above mentioned kind are known and are the result of advances in the underlying technology which have permitted miniaturization of necessary electronics. The ultrasound industry continues to reduce the size and weight of available diagnostic equipment. The smaller and lighter equipment has improved portability however existing portable ultrasound technology is not without its limitations.

One shortcoming of some portable systems is that the need to hold onto or otherwise manipulate the equipment with both hands during use hinders the ability of a person to productively use the system in some situations. For example, document U.S. Pat. No. 5,590,658 discloses an ultrasound system including a lap top computer with the capability of displaying ultrasound images obtained with a handheld scan head. With this kind of system, in order to ensure the display is visible at all times during acquisition of the ultrasound signals with the scan head, the user must either hold the lap top computer, or change the position of the lap top to face the user who may be moving around to position the scan head in a suitable location during the imaging session. As a result, the system user's hand not being used to manipulate the scan head is often nonetheless occupied and not always free to assist with the medical procedure.

The portable ultrasound systems disclosed in U.S. Pat. Nos. 5,722,412 and 5,738,099, are of the kind so called "hand held" and integrate the display and the transducer in the same unit. While in essence permitting the scan head and the display to be held in one hand, these systems suffer from a different shortcoming. In particular, in order for the ultrasound user to get the desired view, it is frequently necessary to move the transducer repeatedly to different regions of the patient and to different orientations. In so doing, the displays of these systems may not be sufficiently visible to a user unless that user moves about, possibly into awkward positions.

A further kind of portable ultrasound systems is disclosed in document US 6 126 608. This kind of devices comprise a scan head and a main unit. Circuitry for forming the image based on the ultrasound signals collected by the scan head. A display screen which is carried by the main unit and on which the ultrasound image is displayed. The main unit is relatively small and is provided in combination with an attachment member which is mountable around a forearm of the system user such that said display is visible to the system user during use. The said attachment member extending toward a hand of the system user at the end of the forearm and comprising a digit-accommodating opening.

Although this ultrasound system overcomes the shortcomings of the above mentioned known systems, by leaving the hand free to carry out help functions during imaging, permitting at the same time to have the display always optimally directed against the user, so that the said user can have the best view of the display, there are still shortcomings which consist principally in the fact that:

The display is very small in size.

The hand at the arm to which the main unit is attached cannot carry out any task or function connected to the device such as controlling the device by means of control buttons or the like. These tasks must be carried out by the hand carrying the probe so that in certain situations the scanning process must be interrupted or even aborted due to the fact that some controls has to be activated on the main unit.

As a general drawback of the increasing miniaturisation trend of the ultrasound systems consists in the fact that the said portable systems are limited ot certain specific applications of the ultrasound imaging technique and that in order to have systems being able to carry put different kinds of applications of the ultrasound imaging technique bigger and less portable devices are still needed.

Thus, it would be desirable to provide a portable ultrasound system which overcomes these and other shortcomings of the prior art indicated above.

The present invention provides a portable ultrasound system of the kind disclosed above according to claim 1.

The main unit case preferably has the form of a flat parallelepiped. In particular it has two larger sides which form the front and the back side and which are connected by smaller sides being the lateral sides.

The front side is preferably formed substantially entirely by a so called touch screen.

So preferably no key or button or other kind of control are provided on the sides of the case of the main unit.

In a particular embodiment the front and the back sides of the case of the main unit are rectangular.

The main unit comprises all of the hardware and peripherals of the ultrasound system needed for driving the ultrasound probe and for generating, processing and storing the images. Further hardware is provided which is of general purpose such as communications units with other devices, networks or other external peripherals. The transducers which are housed in a probe together with some electronic components as it is used in the current traditional ultrasound probes.

Advantageously the elongated member forming the handle is provided at a distance from a peripheral edge of the case of the main unit which is on the opposite side of the peripheral edge of the said case which is nearer to the said elongated member, the said distance being not longer than the average distance from the palm of the hand to the hollow of the elbow.

According to still a further improvement, in order to adjust the said distance of the elongated member from the peripheral edge on the side of the case opposite to the peripheral edge to which the said elongated member is nearer, the arms supporting the elongated member are secured to the case of the main unit and/or to the ends of the elongated member in a swingable way and particularly in a swingable way around an axis which is parallel to the axis of the elongated member.

According to a further improvement (not claimed) the elongated member is hollow and forms a case for housing at least part of the circuitry of the main unit.
In particular the elongated member forms the housing for a battery pack.

According to a first alternative (not claimed) the elongated member is in the form of a case for housing interchangeable battery packs, an opening being provided in the elongated member which allows the battery packs to be extracted from or inserted in the said elongated member.

A second alternative (not claimed) provides that the elongated member form an enclosure for the battery packs and the electric wires from in which enclosure the battery packs being fixedly mounted.

In both cases the said elongated member being at least partly hollow and forming a chamber, which chamber has an open side and a door for closing the said open side. Furthermore in the chamber there are provided electric contact which cooperate with the electric contacts of a battery pack having predetermined dimensions and a shape corresponding to the dimensions and to the shape of the chamber.

In this case the electric cables for feeding the power to the circuitry inside the case of the main body passes inside one or both of the two lateral arms and from then inside the case of the main body.

A further variant (not claimed) provides that the elongated member has several separated chambers each one of this for at least one or a number of battery packs which is limited relatively to the total number of battery packs which can be housed in the elongated member and each chamber having a opening for inserting and extracting the corresponding battery pack or battery packs and having also removable means for closing the said opening, each of the said chambers being provided with contact terminals of a power feeding line which is connected to the power input of the main unit by means of conductors passing through one or both of the arms linking the elongated member to the case of the said main unit.

It has to be stressed out that with the term battery pack, in the present description and in the claims it is meant a rechargeable or non rechargeable battery formed by one piece or a group of said pieces which are electrically connected and integrated in a power unit having a certain number of said singular pieces electrically connected so to have a certain voltage and a certain power and endurance.

According to still a further variant (not claimed) the elongated member has at least one chamber in which a power feeding circuit is housed, the said circuit having means for being connected to an external power network or source and an output which is connected to the power feeding line of the main unit which line is provided in the elongated member.

Advantageously (not claimed) a battery pack recharger is also provided which is fed by the power circuit and which is connected to the battery packs in the elongated member by means of the power feeding line present in the said elongated member or to a separate recharging line which is provided in the elongated member and which branches in each one of the chambers housing the battery packs in which chambers the recharging line has terminal contacts which are electrically connectable to the recharging inputs of each of the battery packs.

It has to be noted that as it is used for example for the nootebook computers the chamber for housing a battery pack and the battery pack itself have each one a part of an electric multipolar connector which parts cooperates one with another for generating the electric connections between the inputs and outputs of the notebook and of the battery pack when the said battery pack is inserted in the chamber.

According to a further improvement, the elongated member carries at least one or more control buttons or keyes, which are connected to the circuitry of the main unit for generating and sending commands to the said main unit and activating or deactivating functions of the ultrasound system.

The elongated member forming the handle is oriented advantageously parallel to a peripheral edge of the display. This allows to carry the main unit easily in the correct orientation of the display for the user.

According to still a further improvement, the case of the main unit can be carried easily either in a so called portrait or in a so called landscape orientation of the display, this means according to two orientations of the display which are rotated of 90° one with respect to the other around an axis of rotation which is perpendicular to the display and passes through the centre of the display area.

When the display is rectangular in shape this means that the display is oriented with the longer edges along an horizontal direction (landscape orientation) in a first orientation and with the longer edges in a vertical direction (portrait orientation) in the second position.

In the said first orientation the elongated member forming the handle of the case of the main unit is also oriented along an horizontal direction, while in the said second orientation the elongated member is oriented along a vertical direction.

In order to have a secure support of the main unit case in both the said first and second orientations, the length of the case of the main unit in the direction parallel to the longitudinal axis of the elongated member is less than twice the said average distance from the palm of the hand to the hollow of the elbow.

This features ensures that when the main unit is held in a position with the handle, i.e. the elongated member, in a vertical plane, the case is balanced and there is not a mayor contribution of the weight in the part of the case protruding over the zone at which the hand grasps the elongated member.

In order to further facilitate the carrying of the main unit, reducing the stress or work of the hand to firmly block the case grasping the handle and so allowing to use the said hand for pressing the keys of buttons arranged on the elongated member, the invention provides an ultrasound system with one or more of the features disclosed above having also an attachment member for said main unit case, said attachment member being mountable around a forearm of the system user such that said display is visible to the system user during use.

In its simplest form the attachment member is formed by at least one strap secured to the back side of the case of the main unit, i.e. the side oriented towards the forearm of the user, and which strap has two ends which carry means for securing the said two ends one to the other and for tightening the strap around the user forearm.

The straps are provided in an intermediate position between the elongated member and the side of the case of the main unit opposite to the one which is nearer to the elongated member.

Furthermore the at least one strap can be secured to the back of the case of the main unit in such a way as to be rotatable around an axis perpendicular to the said back side of the case of the main unit in such a way that the main unit can be rotated in one of the said first and second orientations of the display when the strap is tightly secured to the user arm.

According to a further development, the attachment member is formed by a combination of a strap and an anatomic saddle-like contact part against the fore arm. In this case, the said saddle-like contact part is secured to the back of the case of the main unit in a rotatable way around an axis perpendicular to the back side of the said case.

Furthermore means may be provided for releasable stopping the said saddle-like contact part in one angular position of the at least two angular positions corresponding to the above mentioned first and second orientations of the display.

This can be obtained by means of elastically releasable stopping means which are mounted in housings provided in the back side of the case of the said main unit or in the surface of the said saddle-like contact part adhering to the said back side of the case, while the said means have one or more teeth or projection that are forced in a position protruding out of the surface of the said back side and can be pushed back in a position not protruding for the said back side, the said teeth or projections cooperating with notches provided in the surface of the saddle-like contact part adhering the said back side and the said teeth or projections being placed on the same a circular path coaxial with the axis of rotation as the notches.

According to still another embodiment (not claimed), in order to be able to use the device for longer time without any connection to an external power source, in combination with the case of the main unit further additional power units can be provided consisting in a case for further battery packs. The said case and the case of the main unit have releasable mechanical connection means and releasable electric connection means between the power unit out put line and the main unit power input line.

A first embodiment (not claimed) provides and additional elongated element as the handle which can be mechanically secured to the handle and which is in the form of an enclosure in which the battery packs are stably housed or in the form of a case for housing battery packs in way that they can be extracted from and inserted in the said case for being substituted with charged battery packs.

According with a further embodiment (not claimed) which can be provided in combination or separately from the said first one, the elongated member forming the handle is secured to the case of the main unit in a releasable way, while two or more different elongated members are provided which are different in size one from the other and particularly which have different cross-sections so to be able to house a different number of battery packs. The releasable attachment of the elongated members has the same shape and dimensions for every elongated member so that each one of the different elongated members can be secured to the case of the main unit.

The means for releasably securing the elongated member to the case of the main unit can be of every kind and lies within the general knowledge of the skilled person. Particularly these means can be releasable snapping means, screw-connections, or similar.

As a further feature (not claimed) which can be provided separately or in combination with each one of the features disclosed above, the power unit can be part of a docking unit, which is formed by a case having the same form in plan view as the case of the main unit and having a certain thickness.

The docking unit can be the housing not only for supplementary battery packs but also for further peripherals such as: special computing circuitry, storage devices, reading/writing devices of portable storage media like floppy, CD-ROM, DVD-ROM or similar, modems or other communication hardware, ports for connection of peripherals, like USB, Firewire, parallel and/or serial ports, hardware and ports for the connection to a network, and other devices.

Similarly to the above mentioned elongated member of the handle, the case of the docking unit and the case of the main unit are provided with means for securing one to the other in a releasable way and this mechanically and also electrically.

So also in this case releasable snapping means can be provided or other particular means which ensure a rapid attachment and a rapid separation of the case of the main unit to or away from the case of the docking unit in combination with connectors for the power feeding lines between the power sources in the docking unit and the circuitry in the main unit and for the communication lines of the peripheral and other devices housed in the docking unit and listed above.

As a further alternative the further battery packs and/or the further hardware may be housed in the anatomic saddle-like contact part which has been disclosed above. Also in this case, since the said saddle-like contact part can be releasably secured to the back side of the case of the main unit, a set of different saddle-like contact parts can be provided which differs one from the other relatively to their dimensions so to be able to house a different number of battery packs and/or one or more different additional hardware units.

The said saddle-like contact part can also be provided as unit formed by several parts which can be mounted one to the other when needed. In this case for example, an additional case like element can be provided to be secured in an intermediate position between the saddle-like contact part and the back side of the case of the main unit, while advantageously the said additional case like element has on one to be connected to the backside of the case of the main unit the same connection means as the side of the saddle like contact part, while on the side to be connected to the said saddle-like contact part it has the same connection means as the ones provided on the back side of the case of the main unit. This construction allows also to provide more than one additional case like element which can be secured one to the other and in an intermediate position between saddle like contact part and back side of the case of the main unit.
It has to be understood that the above features can be provided alternatively or in any combination or subcombination one with the other depending on the configuration needed for the ultrasound diagnostic system.

Thanks to the above conditions, the portable device can be configured in such a way as to set a certain endurance of the power source, the possibility to carry out certain tasks and the limiting of the weight in view of the power source endurance and of the tasks which can be carried out.
Typically the endurance of the power source, i.e. for the battery in use is about four hours, while the weight is not lower than 5,5kg.
In its lightest configuration the weight is about 3kg, but the endurance of the power source is limited to about one hour.

Since in the portable ultrasound systems, the endurance of the electric power and the weight are important limitations in comparison to the conventional sonographers a further feature (not claimed) can be provided in any combination or subcombination with the above disclosed features and even separately. The ultrasound systems can be used for carrying out different imaging tasks in relation with diagnostic and/or surgical or mini invasive surgical applications. While conventional echographic systems have the capacity of carrying out everyone or a great part of the above tasks, due to the limits disclosed above the portable device are also limited in relation to the different possible kind of application they are able to carry out.

In the known portable ultrasound systems of the so called "hand held" or "nootebook" kind, in order to reduce power consumption, it is known to limit the feeding of power only to the electronic units which are needed for carrying out the tasks connected to certain specific applications which has been selected by the user. In a case the user interface is always completely active.

According to a further embodiment (not claimed), the portable ultrasound system of the above kind has in the case of the main unit a completely graphical user interface. In this case the screen being of the touch screen kind being able of receiving commands by touching the screen and the different control keys, buttons, switches and cursors being in the form of graphic symbols representing the said keys, buttons, switches and cursors. Selecting means are provided for selecting at least one specific application within a list of application which are available on the portable echographic system, means being provided for loading and executing a specific graphic user interface program which generates on the screen the images of only the keys, buttons, switches and cursors that are needed for carrying out the tasks related to the selected specific application.

Furthermore, according to a further feature (not claimed), in the portable echographic system different electronic units for carrying out specific tasks are substituted by a central processing unit able of executing different programs and of carrying out different tasks, at least a memory being provided in which there are saved different programs for controlling the central processing unit in order to carry out different specific tasks related to the functions of an echographic system, one or more of the said different programs for carrying out specific tasks being loaded by the central processing unit and executed by it when a certain specific application has been selected by the user.

Advantageously (not claimed) a main control program can be loaded and executed by the central processing unit which program prints on the display screen the list of applications available with the said portable system and a further graphics user interface comprising the icons of keys, buttons, switches, cursors or similar control devices related to general functions, such as a menu for addressing the specific diagnostic or surgical applications and or menus for addressing configuration and adjustments options for the device which are of general purpose, and/or menus for carrying out maintenance or managing tasks of the apparatus and of the devices integrated in it such as file manager routines, memory drives control and maintenance routines, electronic units configuration and diagnostics, driver upgrade, system upgrade and further typical tasks needed for managing and for maintaining the efficiency of a computer system.

In particular as soon as the apparatus is started all the touch screen drivers and the menus graphic interface are loaded and executed. The said main control program addresses then depending on the choice made with the help of the menus the programs for carrying out the specific tasks related to the applications or configuration and adjustment options chosen by the user.

According to a further advantageous feature of the present portable echographic system (not claimed), a further program is saved in the memory and is available for being executed by the central processing unit, which program consist in a application specific tutorial for the user which is already provided in the echographic system in a ready to start condition.

In this case the main control program prints on the screen a graphic user interface with a key icon which addresses the said tutorial program and which key when touched by the user determines that the main control program loads the tutorial program and to execute the said program by the central processing unit.

Here (not claimed) also a main tutorial control program can be executed which loads a graphic user interface showing a list of options each one of which can be highlighted and chosen by means of the touch screen interface and which causes the corresponding specific program to be executed and the corresponding graphic user interface to be loaded and printed on the touch-screen display. In order to spare electric power and maintain the weight limited without affecting the computational power of the system needed by the hardware for executing the programs and carrying out the tasks of the system, according to a further feature (not claimed), in the present ultrasound diagnostic system a part of the tasks to be carried out by the central processing unit (CPU) are carried out by the graphic processing unit (GPU). In the ultrasound devices the GPU is not subjected to high operational stresses because the image processing itself is not a heavy operation. Computational power is normally needed to extract the image data from the signals received. Thus the GPU can be used as an aid to the CPU also for carrying out non typical tasks for the GPU. In the present invention the portable ultrasound system has a scan converter which is formed by a software to be executed by a processing unit while the graphic processing unit is used as the processing unit for executing the scan converter software.

The above features and the related advantages will become clear form the following description of preferred embodiments which are illustrated in the annexed drawings, in which:
Figure 1 is a perspective view of the main unit of an ultrasound system according to the present invention in its condition laying on a table and in its simplest configuration as a tablet PC.
Figure 2 illustrates the main unit of figure 1 in the condition being transported as a brief case.
Figure 3 illustrates a first condition of holding the main unit during use and corresponding to a panoramic orientation of the screen.
Figure 4 illustrates a second condition of holding the main unit during use and corresponding to a portrait orientation of the screen.
Figure 5 to 8 illustrates several views of the combination of the main unit and of a main unit table support which allows to position the main unit such that the screen is positioned approximately in a vertical position, while in fig 8 the main unit is separated from the support.
Fig. 9 illustrates the backside of the main unit to which a saddle-like contact part is attached for securing the main unit to the arm and leaving the hand free from grasping the handle.
Fig. 10 illustrates a variant embodiment in which the elongated member forming the handle is doubled by attaching a second elongated member to the first one.
Fig. 11 illustrates a variant embodiment in which the main unit is attached with its back side to a docking unit.
Fig. 12 and 13 illustrates a variant embodiment in which the elongated member together with the two supporting arms are releasably secured to the case of the main unit.
Figure 14 and 15 illustrates the same views as figures 12 and 13 bit with an elongated member having a thicker cross section than the one of figures 12 and 13.
Fig. 16 illustrates a particular of an example of releasable connection means which can be used for attaching the different add-ons disclosed in the previous figures.
Fig. 17 illustrates an example of releasable electric connectors which can be used in combination with the said releasable connection means.
Fig. 18 is a block diagram of the software and hardware structure of the portable ultrasound system.
Fig. 19 and Fig. 20 illustrates an example of a particular embodiment of the connector which secures the elongated member to the case of the main unit and also an example of the therein integrated conductors for feeding the power signal to the main unit and or for feeding other electric signals generated for example by keys or buttons or similar devices provided on the elongated member.
Fig. 21 is a schematic example of the lay out of the elongated member which elongated member is void inside and has three chambers one of which is for the power feeding circuit for connecting the system to an external power source such as the electric network and also for a recharging unit for the battery packs while the other two chambers are for housing each one a battery pack.
Figures 22 to 25 are examples of different GUI (graphic user Interface) for different applications to be carried out by the ultrasound system and which graphic interfaces are generated by a software/hardware structure as disclosed in figure 18.
Referring to figures 1 to 4, a portable ultrasound system comprises a main unit and a probe 2 which is connected to the main unit by means of a cable 5.
The probe 2 and the cable 5 can be connected to the main unit by means of usual connectors, which are not illustrated in detail since these connectors are known and widely used for connecting ultrasound probes.
The main unit comprises the hardware and software and the peripherals which are necessary for driving the probe 2 for transmitting ultrasound beams in a body under examination and for receiving ultrasound beams from the said body and which are necessary for processing the received signals in order to generate diagnostic images of the inner parts of the body under examination.

The hardware of the main unit is housed in a case 1 which has the form of a so called portable tablet PC. In particular the case is formed by a thin parallelepiped case having a front and a back side which are the largest sides. The front side is formed approximately entirely by a flat screen, particulalrly a LCD touch screen, 101, such as a LCD screen.

In the example illustrated the front and the back sides are rectangular. Although this is the preferred embodiment, the invention is not intended to be limited to the present rectangular form.
The lateral sides connecting the front and the back sides are the smaller sides of the case.

Two arms 3 protruding from two corners at the ends of one lateral side carry at their ends an elongated member 4. These two arms departs from the opposite ends of one lateral side or from an edge of the back side or from two opposite lateral sides and are oriented in the direction of the back-side of the case 1 and protrude backwards of the said back side, terminating at a certain distance from the back side. Thus the elongated member lies at a certain distance backwards relatively to the back side of the case 1 of the main unit.

Preferably the two arms 3 have identical lengths, so that the elongated member is positioned parallel to the back side of the case and also parallel to one edge of the said back side.

In the present example, the elongated member is parallel to the longer side of the rectangular back side of the case.

Furthermore, the two arms are inclined in such a way that the elongated member is carried also in a position in which the said elongated member is laterally spaced from the lateral edge of the back side of the cage, so that the said elongated member does fall outside the boundaries of the back side.

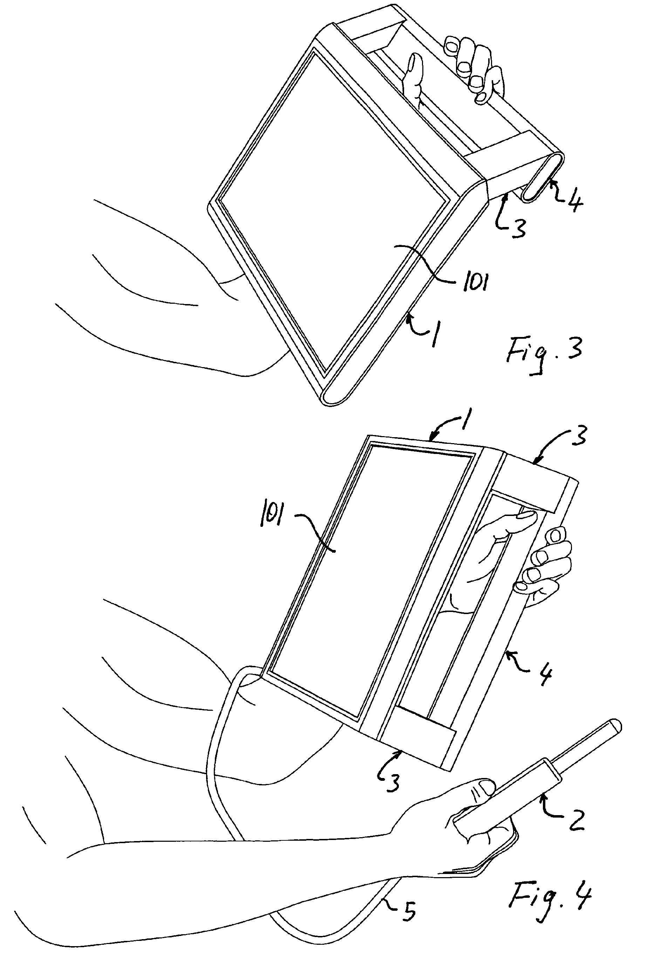

The elongated member has a cross section which is such that it can grasped and held by one hand as illustrated in figures 3 and 4.

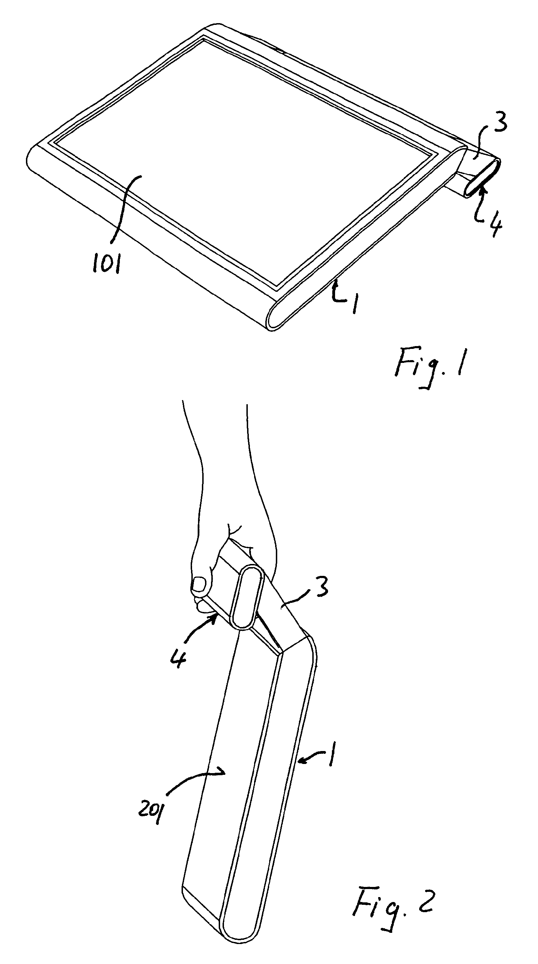

As it appears from the figures 1 to 4, the particular configuration of the elongated member and its position relatively to the case 1 of the main unit allow that the said handle has several functions. As illustrated in figure 1, the elongated member 4 has the function of holding the case inclined when it is placed on a table, so that the screen at the front side 101 of the case is inclined in a way ameliorating the sight of the screen. As illustrated in figure 2, the elongated member serves as a handle for carrying the ultrasound system in a way similar to a briefcase.

As illustrated in claims 3 and 4, the elongated member 4 serves as a handle for blocking the case of the main unit in place and supporting the weight of the said case by means of the same arm the hand of which is grasping the elongated member 4.

According to a preferred embodiment the length and the width of the case 1, i.e. the lengths of the edges of the front and back side of the case 1 are such that they are not longer as the mean length of the fore arm approximately from the wrist to the hollow of the elbow.

This allows when the front and back sides are rectangular that the case 1 can be held either in the portrait or in the landscape orientation of the rectangular monitor. When the configuration of the main unit is such that the elongated member is positioned parallel to the longer side of the front and back side of the case, than when the hand holds the elongated member horizontally or approximately horizontally, the monitor has a landscape orientation and the longest side of the case opposite to the one at the elongated member rests on the forearm.

In figure 4 the hand holds the elongated member 4 in a vertical plane and the monitor has a portrait orientation, which means with the longest side in a vertical direction or in a vertical plane, while the lower shorter side of the case which is parallel to the lower shorter edge of the monitor rests on the forearm. In the configuration of figure 3 the arm is oriented along a direction which is approximately parallel to an axis which is perpendicular to the elongated member and parallel to the back side. Preferably the fore arm is approximately coincident with the central axis of the back side which axis is perpendicular to the elongated member 4.

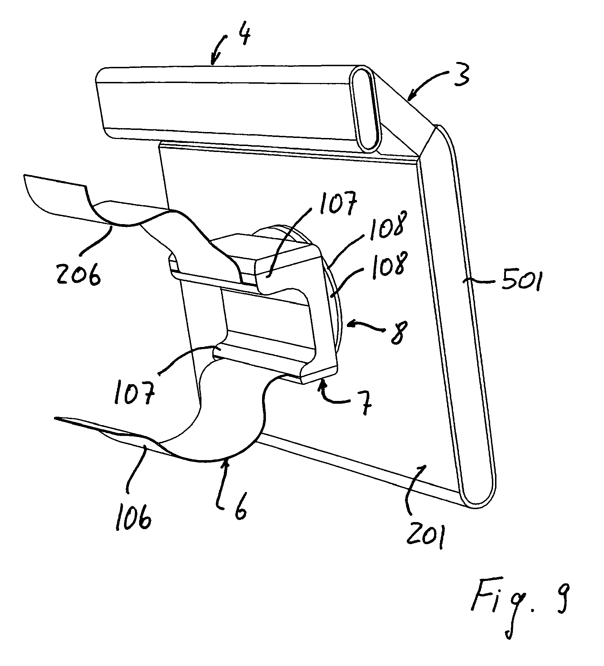

In figure 4, the arm is oriented approximately along a diagonal of the back side of the case 1.
As it appears from fig 9, according to a variant, the case 1, may be provided on its back side 201 with means for securing the case to the fore arm.

The simples means that can be provided are one or more parallel straps that can be tightened around the forearm. For example a strap 6 having a certain width has two ends 106, 206 which carry means for connecting together the said two ends and for tightening the strap. This means can be for example of the kind of the so called velcro tape, one part of which is provided on one end 106 and the other part of the velcro strap is provided on the other end 206 of the strap 6.

Other kinds of connecting and tightening device can be provided as for example the means used for the straps of the diving masks or for the belts of the backpacks.

Because the back side 201 of the case 1 is flat, according to a further feature, the strap or the straps 6 are provided in combination with an anatomical contact part 7. This part is illustrated in figure 9 and is of the saddle-like kind, having an U shaped cross section and a form and dimensions allowing the forearm to fit therein at least for a part of its cross section. The ends of the strap 6 departs each one from one of the arms 107 of the U cross section of the saddle-like contact part.

According to a further feature, the said saddle-like contact part 7 is secured to the back side 201 of the case 1 by means of a device allowing its rotation along an axis perpendicular to the said back side 201. In particular the rotation axis of the said saddle-like contact part coincides with the central axis perpendicular to the said back side 201 of the case 1. Many ways can be provided for allowing the saddle-like contact part 7 to rotate. One example of device 8 for allowing the rotation of the saddle-like contact part 7 is illustrated in figure 9 and has two circular plates 108 secured together at their centre in such a way that the two plates can rotate one relatively to the other. One plate being secured to the back side 201 of the case while the other plate is secured to the saddle-like contact part 7. Further more between the two plated there can be provided means for provisionally stopping the two plates in certain angular positions one relatively to the other. This means are not illustrates since many solutions are possible which lie within the opportunity choice of the skilled person. As an example, one plate could be provided on a circular path coaxial with the axis of rotation with one or more notches or cuts which are distributes along the said circular paths, while the other plate is provided on the same circular path with one or more balls, which are housed in a hole and which are pushed permanently with a certain force against the other plate by mean of an elastic element such as a helical spring. When during relative rotation one with respect to the other one of the balls is coincident with one of the said notches the rotation is stopped and can be prosecuted only by applying a certain release force, which is needed in order to push back the ball in the hole and free again the movement of the plates.

Thanks to the fact that the case can be secured tightly to the fore arm, the hand must not anymore grasp tightly the elongated member and is merely for further ensuring that the device will not slip away and fall down and also for balancing the device on the forearm. Thus the hand can be used to activate one or more keys or buttons or the like which may be present on the elongated member and which can be serve for general purposes or for activating special functions during the execution of a scanning so that the hand holding the probe 2 is not necessary and the examination must not be stopped for entering the command or control needed.

The elongated member is advantageously empty inside at least partially and made in such a way that it forms the housing of one or more battery packs or other hardware.

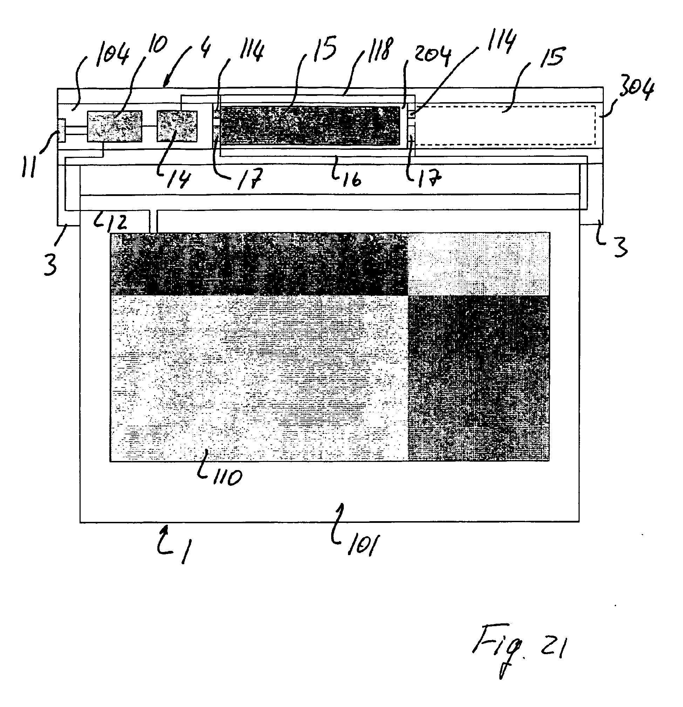

An example of this construction is illustrated in figure 21. The figure is very schematic since the constructive arrangements are matter falling within the technical general knowledge of the skilled person.

Figure 21 represents only one example of several different configurations which are obvious once one configuration is disclosed.

The illustrated elongated member has three separate chambers. Chamber 104 is destined to housing a power feeding circuit indicated with 10 and which is connecter with its input to a connector 11 at one external side, particularly one head side of the elongated member 4. The output of the power feeding unit 10 is connected to the hardware indicated by 110 in the case 1 by means of a power feeding line 12 which passes from the elongated member 4 inside the case 1, by passing through one arm 3 connecting the elongated member 4 to the case 1. In the same chamber 104 or in another separated chamber (non illustrated) a further circuit can be provided which can be for example a battery charger 14. The battery charger 14 can be fed with power by the power feeding unit 10, while its output is connected to a charging connector 114 provided in each of two battery packs housing chambers 204, 304. In this case it is assumed that the battery packs 15 has charging inputs and power outputs which are separated and for which in the corresponding housing 204, 304, there are provided connectors 114 and 17 for automatically connecting a charging line 18 and a power feeding line 16 to the charging input and to the power feeding output of the battery pack 15 when it is inserted in the corresponding chamber 204, 304.

In the present example the two chambers 204, 304 can house each one a battery pack independently one from the other. So the ultrasound system has two so called battery bays in which a battery pack can be inserted independently if a battery pack is present or not in the other bay.

An alternative configuration may consist in the fact that the power feeding unit and the charger are an external device as in the presently known notebook computers, while the chamber 104 is a further battery housing.

The chamber 204, 304 are constructed in such a way to house more than one battery pack 15.

The chambers 104, 204, 304 have an open side through which they can be accessed, A cover can be provided for each of the said chambers. There might be provided that the cover is for closing the open side of all or of part of the chambers.

A further alternative which can be provided consist in the fact that the battery packs 15 are stably housed inside the elongated member 4 and that their cannot be separated therefrom. When the battery packs have to be substituted the entire elongated member has to be substituted. In this case, the elongated member can be secured to the case 1 or to the two arms 3 in releasable manner.

Also the other variant in which the elongated member 4 forms chambers for housing battery packs or hardware and which housings can be accessed for substituting the battery packs or the hardware may be provided with an elongated member which is releasable from the case or from the two arms 3. In this case this may be important for changing the elongated member in order to mount one elongated member which ca house more battery packs or which has different configuration of keys on it or which has different hardware housed in it.

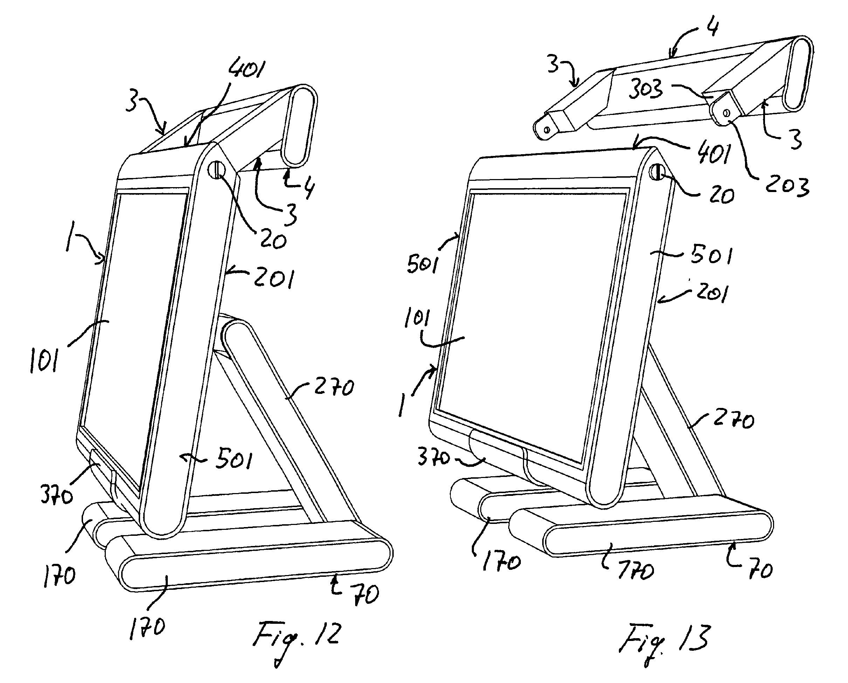

Several different examples are illustrated in figures 12 to 15 and 19 and 10.

In figure 12 to 15, the two arms 3 which support the elongated member 4 in a cantilevered way from the case 1 of the main unit has two terminal protrusions 103 at the end opposite to the one connected to the elongated member. Each protrusion is provided with a passing hole 203. The case 1 of the main unit has at each end of its upper lateral side 401 parallel to which the elongated member 4 has to be held an opening for accessing a pocket which extends parallel to the lateral side 501 which is perpendicular to the one 401 parallel to which the elongated member 4 has to be held and which pocket is dimensioned in such a way that the corresponding protrusion 103 can be inserted therein. The walls forming the lateral sides 501 has a passing hole coinciding with the hole 203 of the protrusion 103. The said hole can pass the pocket from side to side in such a way as to be provided into both walls delimiting the said pocket and one of which walls is formed by the wall of the lateral side 501 of the case 1. The said hole can be threatened along its entire length or only for the part provided in the lateral wall of the pocket which is internal to the case 1. Alternatively the hole 203 in the protrusion 103 is threatened. A screw 20 is provided for being screwed into the hole in the lateral walls of the pocket or in the hole 203 of the protrusion 103 of the arm carrying the elongated member 4.

Relatively to the electric lines for feeding the power signal from the power feeding unit and/or from the battery packs in the elongated member and/or for feeding other signals from other hardware provided in or on the elongated member 4 to the hardware in the case of the main unit, as already illustrated diagrammatically in figure 21, these lines may pass from the elongated member 4 into the case 1 through the arms 3. In the present example of figures 12 to 15, the arms has a bigger cross section than the one of the protrusions 103 and are made tubular. The ends 303 may carry or be conformed as an electric multipolar connector part cooperating with a complementary electric, multipolar connector part which is carried at the lateral side 401 parallel to the elongated member beside the corresponding pocket for the protrusion 103 of each one of the arm 3.

The connector parts will engage each other automatically when the protrusions 103 are inserted in the pockets and are disconnected automatically when the elongated member 4 with the arms 3 is separated from the case 1 of the main unit.

The particular construction of the connector is not illustrated since there are currently available a very large number of electric connectors which can serve to achieve the above function and the skilled person only has to make a choice between the available connectors.

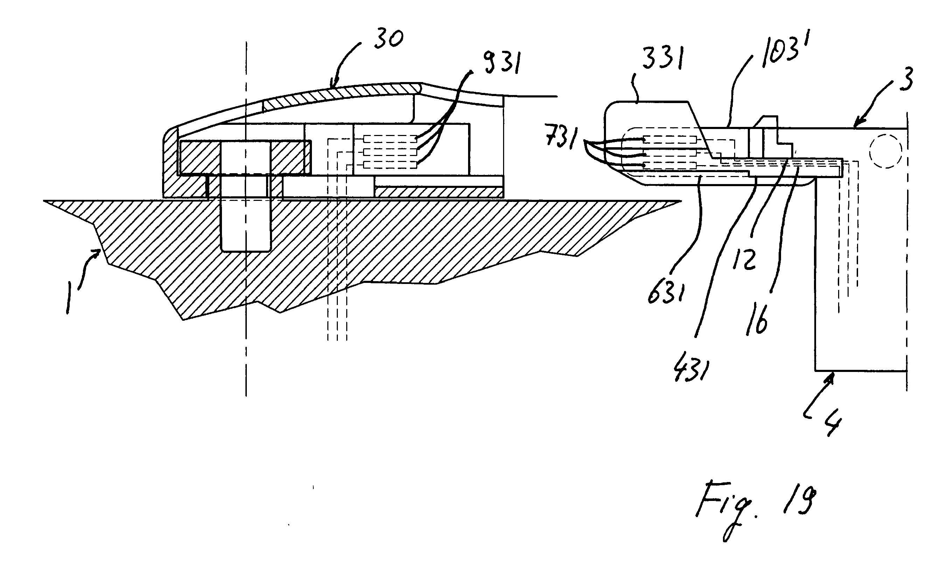

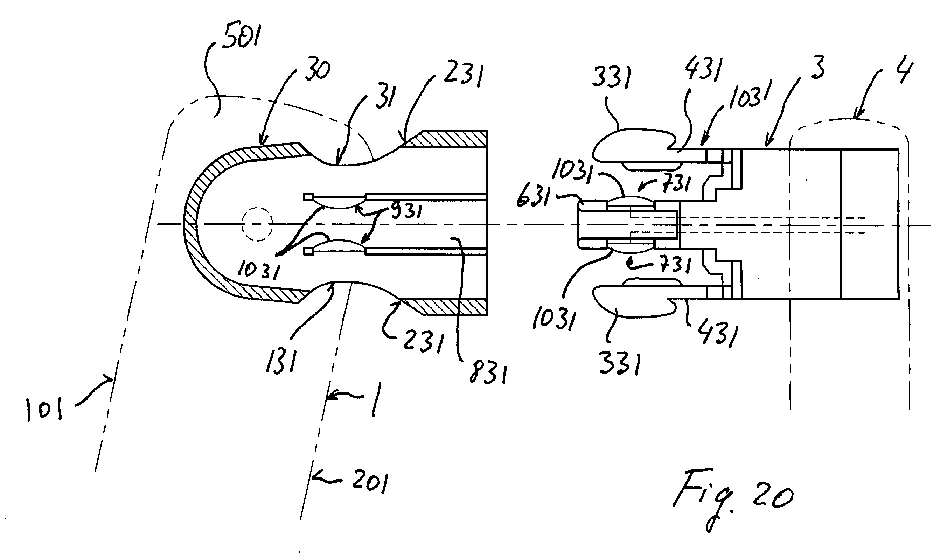

Figure 19 and 20 show a particular which is an alternative to the above embodiment. Here the connection between the arms 3 carrying the elongated member 4 and the case 1 are of the snap in type.

Also in this case there could be plenty of constructions and the one of figures 19 and 20 represents only one example. In this example the connection is of the kind known for example for the belts or for the shoulder straps of the back packs or for the securing straps of the professional fins having an open shoe part.

A pocket like part 30 is secured to each wall of the opposed lateral sides 501 oriented perpendicular to the elongated member 4 and parallel to the arms 3. The pockets has an opening which is oriented towards the arms 3. Each arm 3 has a protrusion 103' which is destined to be inserted in the pocket-like part 30.

The pocket like part 30 has two openings 131 in each of its lateral sides which are perpendicular to the side 501 to which they are secured. The said openings forms an engaging edge 231 for a corresponding engaging tooth 331 which is carried at the end of an elastic tongue 431 of the protrusion 103'. The said protrusion has two elastic tongues 431 which are oriented parallel to the direction of insertion in the pocket 30 and which are eccentric and symmetrically positioned relatively to a central longitudinal axis of the protrusion which is a central axis parallel to the direction of insertion in the protrusion 103' in the pocket part 30. Each elastic tongue 431 is flexible in a direction perpendicular to the said longitudinal central axis and against the said longitudinal central axis, and carry the tooth 331 on its lateral side facing the lateral side of the pocket part 31. In their normal rest position the tongues are oriented in such a way that the rear fronts of the teeth are aligned with the engaging edge 231 of the openings 131 in the lateral sides of the pocket part 30. The end of each tooth is bevelled in such a way as to form a sliding surface of the lateral side of the opening of the pocket part 30 along the said tooth, so that automatically the tongues 431 are bent one against the other allowing the protrusion 103' to enter the pocket like part 30 and slide therein till each one of the two tooth 331 are coincident with the corresponding opening 131 in the lateral wall of the pocket like part and the tongues are free to spring up laterally outwards. Thereby each tooth is automatically engaged in the opening 131 and its rear side is aligned with the retaining edge 231 of the corresponding opening 131.

For freeing the protrusion 103' it is sufficient to press with the hands the teeth one against the other and displace them below the retaining edge so that the protrusion 103' can slip out form the pocket.

A particular multipolar electric connector can be provided in combination of this kind of mechanical connector. The protrusion 103' has a central longitudinal element 631 one of the lateral outer walls or both the lateral outer walls of the said longitudinal element 631 carry several longitudinal electric contacts 731 each one connected to a conductor which passes inside the said central longitudinal element 631 and through the corresponding arm 3 inside the elongated member 4. The pocket like part 30 is provided with a central guide 831 for the central longitudinal element 631, which central guide is a channel like and has lateral walls each of which carry a corresponding number of electric contact 931 which have such a position that each contact will be coincident and will be in contact with a corresponding electric contact 731 on the central longitudinal element 631 of the protrusion 103' when the said protrusion 103' is inserted in the pocket like part 30 in its end position. Each contact 931 is connected to the hardware in the case 1 by means of a conductor passing inside the case 1 through passages in the pocket like part 31 and in the lateral wall 501 to which this part is secured.

Advantageously as indicated in the drawings the electric contacts 731 and 931 are of the kind having an elastic contact terminal 1031 which is urged elastically in a position protruding outwards from the surface of the central longitudinal element 631 and from the lateral walls of the central guiding channel 831 in which the central longitudinal element 631 of the protrusion 103' of the arm 3 slides when the said protrusion is inserted in the pocket like part 30. This protruding elastic contact terminals 1031 can have an arched form so that they are invited to slide one over the other when the electric contacts 731 and 931 comes to slide one over the other.

As it appears from the figures 12 to 15, the present releasable elongated member 4 allows to provide different kinds of elongated elements having the same arms 3 and the same kind of protrusions 103, 103' so that different kinds of elongated elements 4 can be mounted on the case 1.

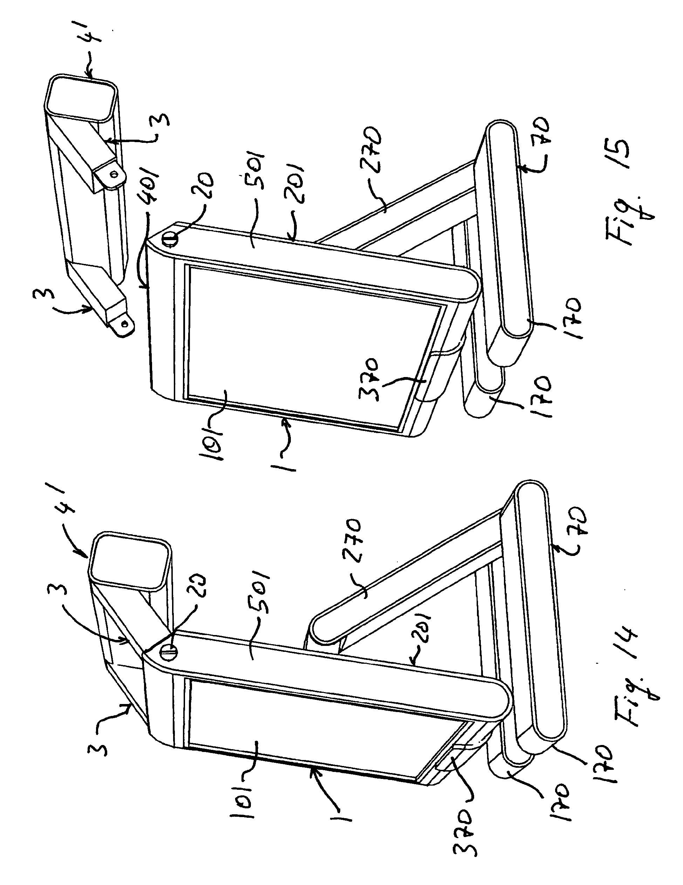

Here for example the two elongated elements 4 of figures 12 and 13 and of figures 14 and 15 differ one from the other in the fact that they have different cross sections. The elongated element of figures 14 and 15 has a cross section which is approximately double in size of the area than the cross section of the elongated member of figures 12 and 13. This allows to have a bigger number of chambers or a bigger chamber for housing more battery packs and/or more hardware in the elongated member 4 and/or also more keys or buttons or commands. When the not a long endurance of the battery packs is needed the thinner elongated element 4 and be mounted on the case. When more endurance or more power is needed the thicker elongated member can be mounted on the case. Also if different additional functions such as for example communication hardware and/or additional driver units for writing and reading memory supports or additional hard disks units or other devices are needed a particularly configured elongated member carrying the said additional devices can be mounted on the case 1.

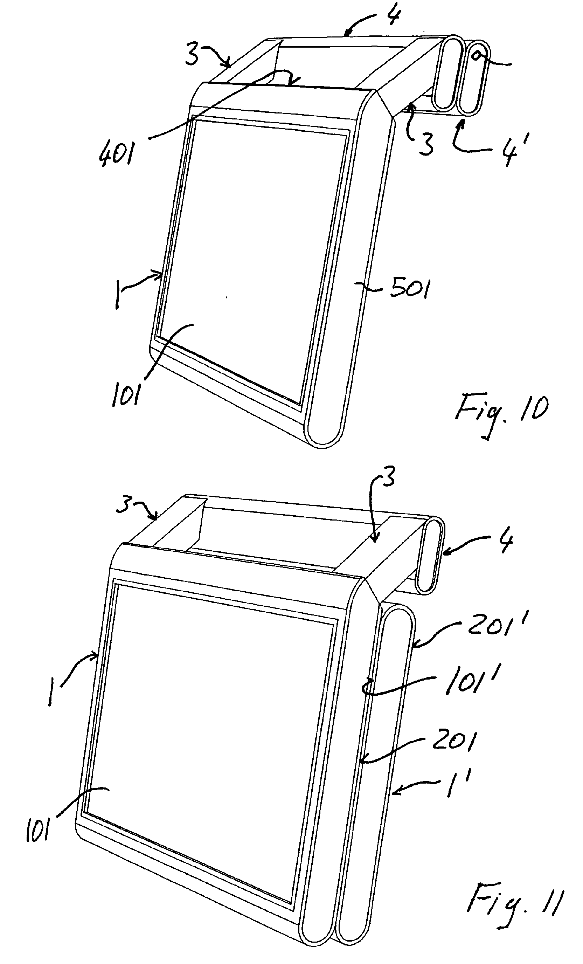

According to a further embodiment of the ultrasound system which is illustrated in fig. 10, a second elongated member 4' can be attached to a first elongated member 4 which is attached to the case 1 by means of the arms 3 either in a stable or in a detachable or releasable manner as described above. The second elongated member 4' serves as a case for further battery packs or further hardware according to the different constructions disclosed above for the first elongated member 4. In this case the power feeding lines and/or other communication lines of the hardware housed in the second elongated member 4' for connecting the battery packs and/or the hardware housed in the said second elongated member to the hardware in the case of the main unit passes through the first elongated member 4. This is done by means of an electric connector of the kind used for connecting a docking station with its notebook computer. In particular a two part connector is provided, a first of the said connector parts being provided on the side of the first elongated member 4 against which a facing side of the second elongate member 4' come into contact when the said second elongated member is attached to the said first one. A second connector part is provided on the said side of the second elongated member facing the side of the first elongated member 4 against which the said second elongated member 4' will come into contact with the said first elongated member 4.

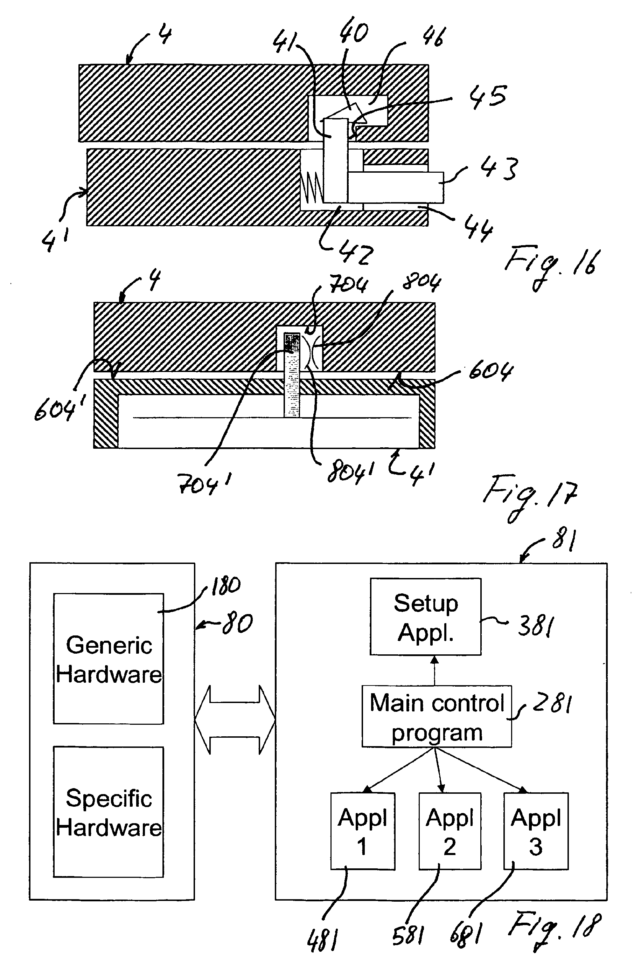

Particularly advantageous is a connector as the one illustrated in figure 17. here the side 604 of the first elongated member 4 has a slot 704 in which a plurality of contact terminals 804 are aligned forming a row of contacts which are oriented with their surfaces of contact to the contact terminals 804' of the second elongated member in a plane perpendicular to the side 604 of the first elongated member 4, i.e. perpendicular to the side of the said first elongated member against which a facing side 604' of the second elongated member 4' is brought in contact when the two elongated member are attached one to the other.

The second elongated member 4' is provided in a position coincident with the said slot with a connector plate 704' carrying a row of contact terminals 804'. The contact terminals 804' are oriented parallel with the contact terminals 804 of the connector part provided on the first elongated member 4 and laterally shifted in such a way that each one of the contact terminals in the slot 704 of the first elongated member 4 will slide against a corresponding one of the contact terminals 804' on the connector plate protruding out of the side 604' of the second elongated member 4'. The contact plate 704' being parallel to the slot and to the surface in the slot 704 carrying the row of contact terminals 804. Each contact terminal 804, 804' can be further formed by a flexible tongue which is arched along an axis perpendicular to the direction of engagement of the contact plate 704' in the slot 704. The arched tongues of the contact terminals 807 in the slot protrudes in the direction of the arched contact terminals 804' on the connector plate 704' of the second elongated member 4' such that the arched tongues of the contact terminals 804 and the one of the contact terminals 804' interfere one against the other, so that each contact terminal 804 and the corresponding contact terminal 804' are pressed elastically one against the other in the engaged condition of the two parts of connector.

When bringing the second elongated member against the first elongated member 4 by aligning the two connector parts the said ones will be automatically engaged one in the other.

The first elongated member 4 can be provided with a further power line for the additional battery packs of the second elongated member 4' or the output of the power lines in the second elongated member are connected to the power lines of the first elongated member 4 so that an extension of the already present power line network is generated.

Similarly the communication lines of the additional hardware housed in the second elongated member can be connected to separate communication lines already provided in the first elongated member 4 and which are dedicated to the said additional hardware or the communication lines provided in the second elongated member 4' can be connected with the communication lines of the first elongated member 4 forming an extension of a communication network which is common to every additional hardware in both the two elongated members 4, 4'.

It is to be noted that not only two elongated members 4, 4' can be attached one to the other mechanically and electrically, but also three or more additional elongated members can be attached in a cascade way one to the other and to the first elongated member.

The mechanical attachment can be made by means of releasable means such as the ones illustrated as an example in figure 16. One of the two elongated members, and in particular the second or further one 4' carries on the side to be brought into contact with a side of the first elongated member 4 a hook 40 which is provided on a pin 41 protruding perpendicularly out of a housing 42 through an opening of the said housing on the side of the second elongated member 4' destined to come into contact with a side of the first elongated member 4. the pin 41 is carried by a cursor 43 which is mounted slidably in a guide 44. The guide 44 being oriented with its longitudinal axis perpendicular to one of the head sides of the elongated member where it is open allowing to the end of the cursor 43 to protrude out of the said opening and being operable by mean of the fingers. An elastic element maintains the cursor 43 in a position in which it protrudes out of the opening of the guide 44 and the hook 40 at the end of the pin 41 is in a position of engagement of a retaining edge 45 which is formed by a undercut in a lateral wall of a notch 46. the notch 46 is provided in the side wall of the first elongated member 4 destined to be brought into contact with a wall of the second elongated member 4' when the two elongated members are attached one to the other. The notch 46 and the hook 40 being provided in coincident positions when the two elongated members are attached one to the other. The hook has preferably a rounded head which helps to be automatically driven for engaging the retaining edge 45 when the two elongated member 4, 4' are brought with their facing sides into contact one with respect to the other.

In order to release the two elongated members one from the other the hook 40 can be disengaged from the retaining edge 45 of the slot 46 by pushing on the head of the cursor 43 protruding out of the opening of the sliding guide 44.

The above is merely an example for demonstrating that mechanical releasable attachment means can be provided for connecting mechanically the two elongated member one to the other.

The second elongated member 4' can have the same form as the first elongated member 4.

A further variant which can be provided separately or in combination with anyone of the previously disclosed combination of features consist in the provision of a second case 1' having the form and the dimensions of the case 1 of the main unit and which has the function of providing further housings for further battery packs and or further housing for further hardware.

Despite the shape and the dimensions which are such that the second case 1' can be releasable attached with its front side 101' to backside 201 of the case 1 of the main unit, similar features can be provided for this second case 1' as the one disclosed for the second elongated member 4'. Indeed the way and the construction which apply for connecting mechanically one case to the other and for generating electric connections of the battery packs of the additional case 1' and or of the additional hardware to the hardware of the main unit housed in the case 1, can be the same as the one disclosed by means of figures 16 and 17 for the second elongated member. Relatively to the construction of the additional case 1' with reference to the housings for the battery packs and for additional hardware similar features can be provided as the ones illustrated and described for the elongated members in figure 21.

The present embodiment further enhances the possibility and flexibility of the configuration of the device relatively to the endurance of the power supply and to the weight.

Similarly top the construction of the elongated member 4, more than only one additional case 1 can be provided each one of the said additional cases carry each the hook means on one side and the notches on the other side so that a second additional case can be attached to the back side 201' of a first additional case which in turn is attached to the case 1 of the main unit.

According to still another feature, the saddle-like contact part 7 can be secured to the back side of the case in a releasable way thanks to releasable attachment means which can be of any kind, being many different attachment means known to the skilled person.

This allows to connect the case 1 to several other supporting devices which enables a correct or optimised positioning of the device.

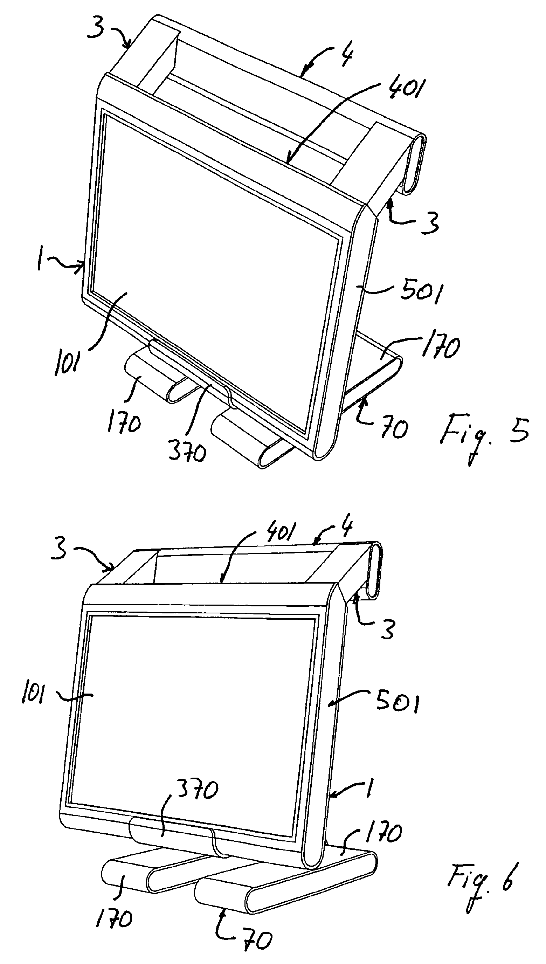

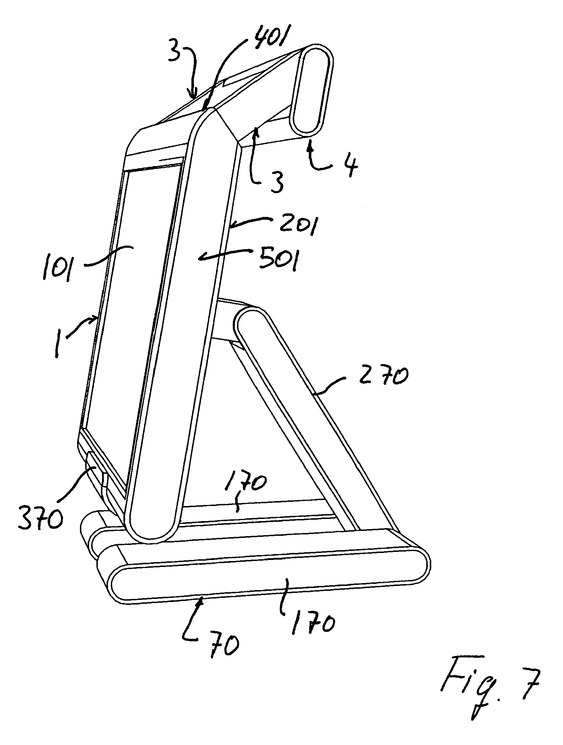

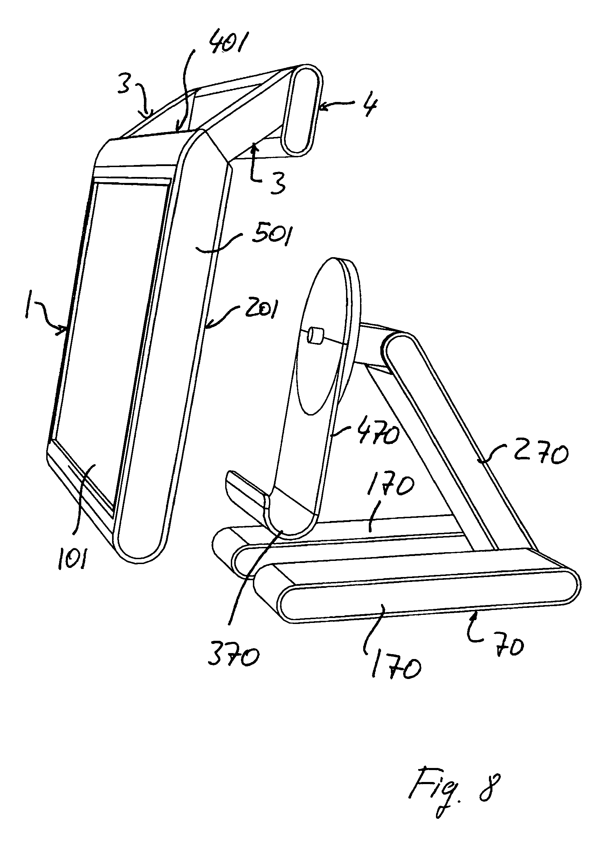

According to figures 5 to 8 and 12 to 15, a table base 70 is provided in combination with the case 1 of the main unit which allows to position the case 1 with the front side formed by the monitor screen substantially in a vertical position. The table base has two horizontal feet 170 which are linked with one of their ends to the end of an intermediate supporting lever 270. at its end the supporting lever 270 carries a back plate 470 to which a lower supporting saddle 370 is secured in a rotatable manner around an axis which is perpendicular to the back plate 470. The lower supporting saddle 370 is provided at such radial distance from the axis of rotation that when the case 1 of the main unit is positioned with its lower horizontal side in the said saddle 370, the axis of rotation is approximately coincident with the central axis perpendicular to the back side of the case 1. the saddle 370 extends till to the said central area of the back side of the case 1 at which it is linked in the rotatable way to the back plate 470 and at which it has releasable connecting means to the back side of the ca 1.

Thanks to this construction while lying on the table base, the case 1 can be freely rotated and the user is able to orient the monitor screen in a landscape or in a portrait position.

The hardware driving the monitor of the main unit can advantageously have automatic means for detecting the orientation of the screen of the monitor relatively to the said landscape or portrait orientation and which means automatically adapts the image visualized on the screen to the orientation of the said screen. This means are known and are for example gravity sensors.

Both in the case of the table base 70 and of the saddle like contact part 7 further box like cases may be provided which can be mounted releasably in an intermedite position between the back side of the case 1 and the side of the table base or of the saddle-like connection part 7 facing the said back side 201 of the case 1. each box like case can carry different hardware or further battery packs. One or more of the said box like cases can be provided in such a way that a row of the said one or more boxlike cases secure one to the back of the other are mounted to the back side of the case 1 and to the said row of box like cases the table base or the saddle like connection part are attached.

Here the releasable way of fixing the intermediate boxlike cases can be the same as the one described above for the two or more elongated members 4, 4' or the two or more cases 1, 1' according to the configuration of figures 10 and 11 and 16 and 17.

According to a further embodiment, the portable ultrasound system of the above kind has a completely graphical user interface. In this case the screen being of the touch screen kind and being able to receive commands by touching the screen at different control keys, buttons, switches and cursors being in the form of graphic symbols representing the said keys, buttons, switches and cursors. Selecting means are provided for selecting at least one specific application within a list of application which are available on the portable echographic system, means being provided for loading and executing a specific graphic user interface program which generates on the screen the images of only the keys, buttons, switches and cursors that are needed for carrying out the tasks related to the selected specific application.

In the portable echographic system different electronic units for carrying out specific tasks are substituted by a central processing unit able of executing different programs and of carrying out different tasks, at least a memory being provided in which there are saved different programs for controlling the central processing unit in order to carry out different specific tasks related to the functions of an echographic system, one or more of the said different programs for carrying out specific tasks being loaded by the central processing unit and executed by it when a certain specific application has been selected by the user.

Figure 18 is a simplified block diagram illustrating this structure. The structure is a typical hardware/software architecture. The hardware 80 comprises generic hardware 180 in the form of a processing section able to memorize and execute control programs. The control programs 281, 381, 481, 581 and 681 are of two kinds. Control programs 281 and 381 are general setup programs such as management programs or hardware configuration or setup programs. Control programs 481 to 681 are virtual ultrasound machines, in the sense that routines are provided which when executed by the generic hardware and if necessary by a specific hardware drive the said generic hardware to carry out specific imaging application oriented functions and activate the specific hardware if it is necessary for some very particular application specific functions. Furthermore each control program drives the touch screen in such a way as to generate on it a graphic user interface which comprises icons representing specific keys, buttons, cursors and other controls that are needed for the specific imaging application to which the virtual machine represented by the said control program is directed. The said keys, buttons, cursor icons and the eventually further icons of other kind of manual controls are activable manually by means of the touch screen in such a way as to operate like the corresponding physical hardware devices.

So for example if only bi-dimensional b-mode imaging is needed the main control program will be provided with a selection/activation function of a corresponding control program which is loaded and executed by the hardware 80. According to the said control program a specific graphic user interface is generated on the screen having active icons of control keys, buttons, cursors and/or other manual control devices for controlling an imaging apparatus capable of only acquiring, processing, visualizing and saving B-mode images.

If for example the application needed relates to the determination of vascular flux, if this specific application is provided in the system, then a particular control program selection and activation button will be available which will charge in the hardware 80 a corresponding control program which relates of a corresponding virtual ultrasound apparatus having the functions of operating in the so called Doppler or Power Doppler-mode, other functions which are not needed will be not present in the virtual ultrasound apparatus specialized for determining blood flux.

This are only two examples that can serve to the skilled person to understand the basic idea of the hardware/software structure of the present ultrasound system. Starting from this the skilled person is able to construct any kind of specific application oriented machine and then the control program for generating the corresponding virtual machine.

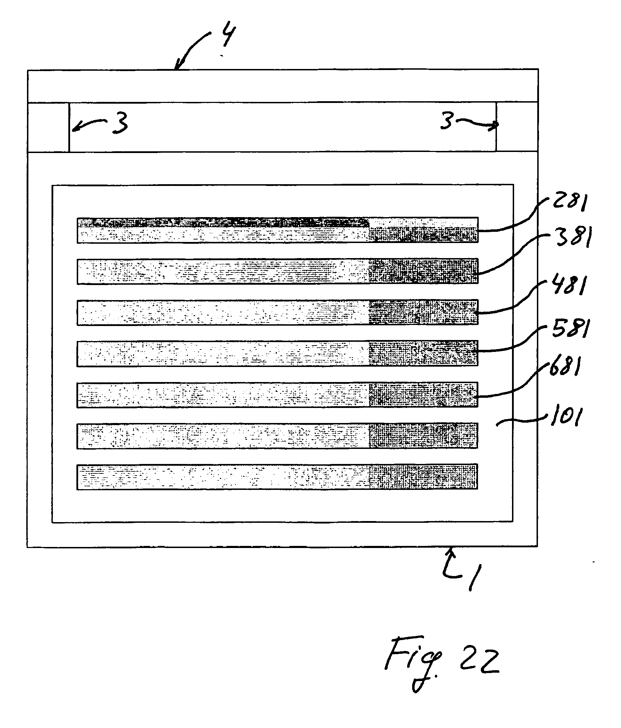

Figure 22 illustrates diagramatically the appearance of an example of the graphic user interface of a main control program.

Here on the touch screen 82 a list of options appears each one of the options being associated to a selection and activation representation of a virtual key or button and each one of the said virtual key or button correspond to an active area of the touch screen which when touched will cause the main control program to address the specific application control program corresponding to the option selected and to load this control program in the operative memory of the hardware which will execute this application specific control program.

In figure 22 seven selection and activation keys are shown as the graphic user interface of the main control program. Two of this corresponds to the setup control programs which are indicated with 281 and 381 in fig. 21 such as the power management setup and the hardware configuration setup. The other five keys are related to five different application specific configurations of an ultrasound system.

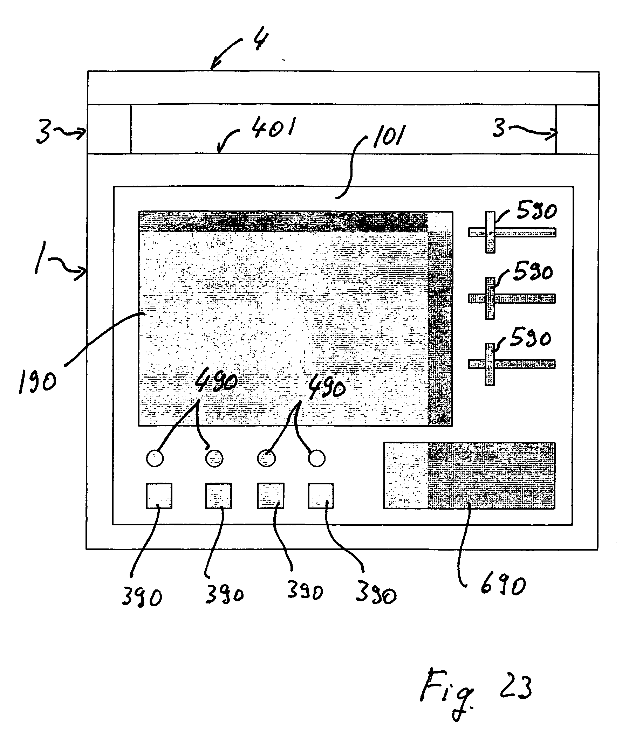

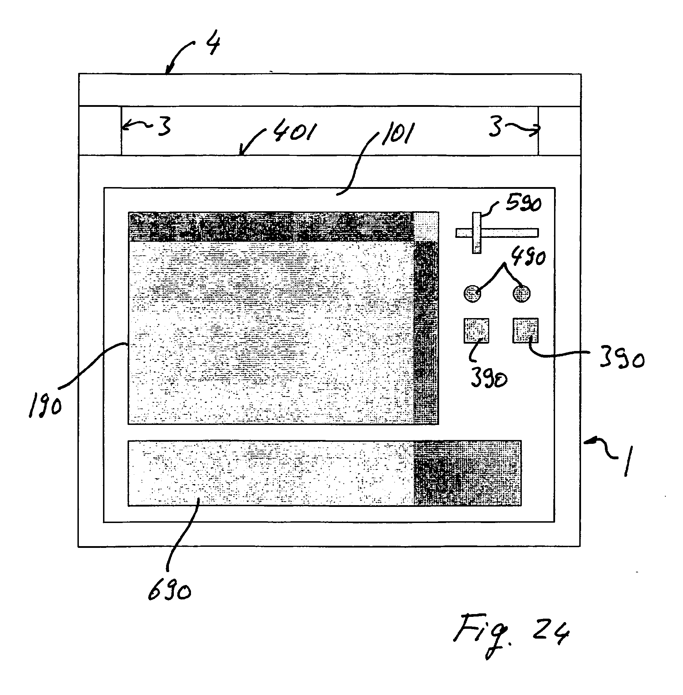

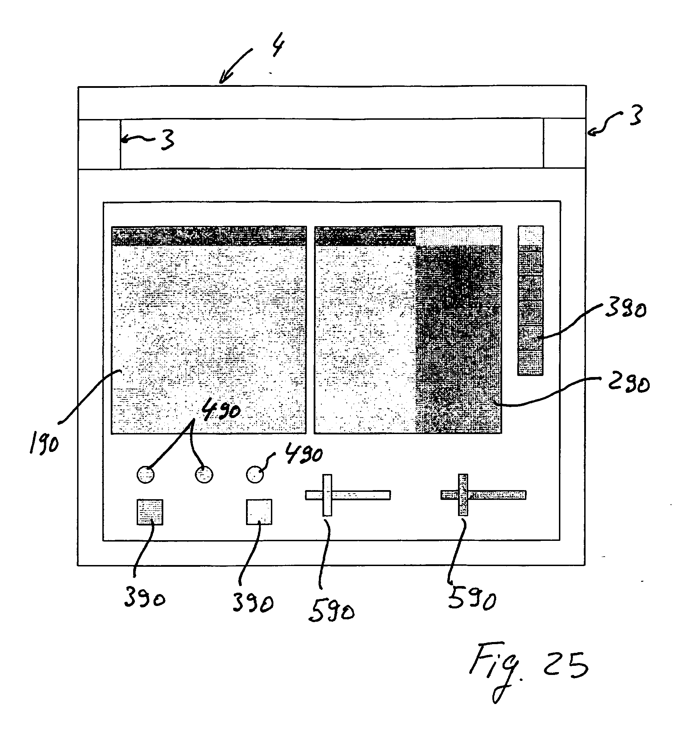

Figures 23 to 25 illustrate three different graphic user interfaces corresponding to three different virtual machines generated by three different application specific control programs such as for example the ones indicated with the numeral 481, 581 and 681 in figure 21.

As it appears clearly the graphic user interface printed on the touch screen comprises different visualisation areas of one or two images indicated by 190, 290; different number of keys indicated with the numerals 390 and different number of signal lights indicated by the numeral 490; different number of cursors indicated by the numeral 590. Further more the keys 390, the lights 490 and the cursor 590 has different positions on the area of the touch screen as also the image visualisation areas 190, 290. Further areas 690 of the screen 90 can be provided for visualizing further informations in the form of an alphanumeric text.

The advantage of the above described architecture are first of all that there is no mechanical control to be activated or driven by the user, so that there are no problem of long term damages of mechanical devices. Furthermore, a hardware configuration would necessitate that a certain number of keys buttons, levers, or other manually activable control means have to be present which for a specific application would be superfluous. Using a graphic user interface the configuration of the manually driven control means can be changed every time in a way which is specific to the imaging application to be carried out so that the configuration of the machine representing the ultrasound system can be limited every time to the provision of the very essential manual controls and thus to the very essential functions.

In addition to the fact that it is possible to spare in the dimensions of the touch screen, in the computational power and in the memory space and thus in the electric power consumption, every graphic user interface corresponding to a certain application specific configuration of the ultrasound system, is very simple to be used and it is also very simple to learn to use the application specific ultrasound system, since the keys, buttons, cursors and the other commands are limited to the ones needed for controlling the application specific machine selected.

In loading the different control programs corresponding each one to an application specific configured ultrasound apparatus, the generic hardware is controlled to carry out functions which in the currently known devices is carried out by dedicated hardware. Only very specific functions will need specific hardware which will have its corresponding software to be correctly driven in combination with the functions carried out by the generic hardware controlled by the control program.

Relating to the above description it has to be stressed out that only some examples are considered among a great number of possible choices.

A possible list of applications for each one of which a specific virtual machine is provided in the form of a specific control program which generates also a dedicated layout of controls which is optimized relatively to the specific application comprises the following applications:
Regional Anaesthesia including Nerve Blocking and Vascular Access;
Veterinary including large and small animals;
Advanced Vascular including Blood Vessel Thickness and Stiffness;
Musculoskeletal including wound View, Sports Medicine and Phlebology;
Esthetical surgery including Fat Thickness and Botox treatment.

The provision of application specific layout of the control interfaces with the apparatus which limits the controls to the ones which are really necessary for controlling the application specific tasks and functions, allows also to generate very simple tutorials which are in the form of executable tutorial programs and which are dedicated each one to a specific application. Particularly each tutorial is a complete demos of the way of using the ultrasound system in the application specific configuration. Furthermore because of the fact that the application specific control programs does not need a huge amount of memory and computational power, each one of the said programs can also be provided with an online held which can print on the screen help messages either automatically or by a request command.

Coming to the detail of the hardware, the generic hardware can be of the kind of a processing unit such as a computer motherboard with integrated video and audio chip, a communication bus with hard drives. A PCI bus for connecting several peripherals, USB and or Parallel and/or serial and/or network communication hardware and ports a solid state work memory for loading the control programs to be executed and a processor. The touch screen is connected to the video chip and has an own processor a so called GPU (graphic processing unit). According to a further feature of the present invention, in order to limit the size and expenses of the hardware and also the weight of the device without loosing computational power, some routines of the control programs representing the virtual apparatuses and which are functional units consisting in a software which drives the generic hardware to carry out the functions of the corresponding unit, are generated so to be carried out by the GPU instead of the CPU (central processing Unit). A particular example consists in the fact that a software scan-converter is provided which is the software version of the currently used hardware scan converter. This software scan converter is executed by the GPU using zero CPU resources. Since the GPU has a considerable computational power which is normally not completely exploited in this case some computational work is passed from the CPU to the GPU so that the CPU is free to carry out other tasks.

## Claims

1. Portable ultrasonic diagnostic system comprising:
a probe for transmitting and receiving an ultrasonic signal to and from a region of interest;
a main unit comprising circuitry in communication with said probe for forming an image of the region of interest based on the ultrasonic signal and;
a display for displaying the formed image;
the said probe being connected to the said circuitry in the main unit by means of a cable connection;
the said main unit having a weight which can be supported by one arm and;
the main unit being provided with a handle to be grasped by one hand;
**characterised in that**
the main unit has a case in the form of a tablet computer, having a front side formed by the display and a rear side;
the said handle being formed by an elongated member which is secured to the case of the main unit by cantilevered arms protruding from the case in a direction of the rear side of the case of the main unit and terminating behind the said rear side so that the elongated member forming the handle extends at a certain distance behind the rear side of the case of the main unit and is nearer to one of two opposite peripheral edges of the said case of the main unit in such a way that when one hand grasps the handle the case of the main unit can rest on the forearm, with the weight of the main unit being carried by the forearm and the hand having only the function of blocking the case of the main unit in position.

2. Portable ultrasonic system according to claim 1 **characterised in that** the main unit case has the form of a flat parallelepiped with two larger sides which form the front and the back side and which are connected by smaller sides being the lateral sides.

3. Portable ultrasonic system according to claim 1 or 2, **characterised in that** the front and the back sides of the case of the main unit are rectangular.

4. Portable ultrasonic system according to one or more of the preceding claims, **characterised in that** the elongated member forming the handle is provided at a distance from a peripheral lateral edge of the case of the main unit which is on the opposite side of the lateral peripheral edge of the said case which is nearer to the said elongated member, the said distance being not longer than the average distance from the palm of the hand to the hollow of the elbow.

5. Portable ultrasonic system according to one or more of the preceding claims, **characterised in that** in order to adjust the said distance of the elongated member from the lateral peripheral edge on the side of the case opposite to the lateral peripheral edge to which the said elongated member is nearer, the arms supporting the elongated member are secured to the case of the main unit and/or to the ends of the elongated member in a swingable way and particularly in a swingable way around an axis which is parallel to the axis of the elongated member.

6. Portable ultrasound system according to one or more of the preceding claims, **characterised in that** the arms supporting the elongated member are telecopically adjustable in length.

7. Portable ultrasound system according to one or more of the preceding claims, **characterised in that** the elongated member carries at least one or more control buttons or keys, which are connected to the circuitry of the main unit for generating and sending commands to the said main unit and activating or deactivating functions of the ultrasound system.

8. Portable ultrasound system according to one or more of the preceding claims, **characterized in that** the elongated member forming the handle is oriented advantageously parallel to a peripheral edge of the display.

9. Portable ultrasound system according to one or more of the preceding claims, **characterized in that** the case of the main unit can be carried easily either in a so called portrait or in a so called landscape orientation of the display, this means according to two orientations of the display which are rotated of 90° one with respect to the other around an axis of rotation which is perpendicular to the display and passes through the centre of the display area;
the case having a rectangular parallelepiped shape and in the said first landscape orientation the elongated member forming the handle of the case of the main unit is oriented along an horizontal direction, while in the said second portrait orientation the elongated member is oriented along a vertical direction and **in that** in order to have a secure support of the main unit case in both the said first and second orientations, the length of the main case in the direction parallel to the longitudinal axis of the elongated member is less than twice the average distance from the palm of the hand to the hollow of the elbow.

10. Portable ultrasound system according to one or more of the preceding claims, **characterised in that** on the elongated member manually activable control means are provided such as keys, buttons, cursors or the like and in order to further facilitate the carrying of the main unit, reducing the stress or work of the hand to firmly block the case grasping the handle and so allowing to use the said hand for pressing the manually activable control means arranged on the elongated member, an attachment member is provided for said main unit case, said attachment member being mountable around a forearm of the system user such that said display is visible to the system user during use and said attachment member having tightening means around the forearm for securing the case to the forearm.

11. Portable ultrasound system according to claim 10, **characterised in that** the attachment member is formed by at least one strap secured to the back side of the case of the main unit, i.e. the side oriented towards the forearm of the user, and which strap has two ends which carry means for securing the said two ends one to the other and for tightening the strap around the user forearm.

12. Portable ultrasound system according to claim 11 **characterized in that** the straps are provided in an intermediate position between the elongated member and the side of the case of the main unit opposite to the one which is nearer to the elongated member.

13. Portable ultrasound system according to claim 11 or 12, **characterized in that** the at least one strap can be secured to the back of the case of the main unit in such a way as to be rotatable around an axis perpendicular to the said back side of the case of the main unit and in such a way that the main unit can be rotated in one of the said first and second orientations of the display when the strap is tightly secured to the user forearm.

14. Portable ultrasound system according to one or more of the preceding claims 10 to 13, **characterized in that** the attachment member is formed by a combination of a strap and an anatomic saddle-like contact part which is fitted onto the forearm, the saddle-like contact part being secured to the back side of the case of the main unit in a rotatable way around an axis perpendicular to the said back side.

15. Portable ultrasound system according to claim 14, **characterized in that** means are provided for releasable stopping the said saddle-like contact part in one angular position of the at least two angular positions corresponding to the said first and second orientations of the display.

16. Portable ultrasound system according to claim14 or 15, **characterized in that** additional battery packs and/or additional hardware is are housed in the anatomic saddle-like contact part.

17. Portable ultrasound system according to claim 16, **characterized in** the said saddle-like contact part is releasably secured to the back side of the case of the main unit, two or more saddle-like contact parts are provided which one or more saddle-like contact parts differ one from the other relatively to their dimensions so to be able to house a different number of battery packs and/or one or more different additional hardware units.

18. Portable ultrasound system according to claim 16 or 17, **characterized in that** the said-saddle like contact part is constructed as a unit formed by several parts which can be mounted one to the other when needed an additional box like case being provided having means for being secured in an intermediate position between the saddle-like contact part and the back side of the case of the main unit, while the said additional intermediate box like case has on one side to be connected to the backside of the case of the main unit the same connection means as the on the side of the saddle-like contact part, while on the side to be connected to the said saddle-like contact part it has the same connection means as the ones provided on the back side of the case of the main unit.

19. Portable ultrasound system according to claim 18, **characterized in that** more than one additional box like case are provided each one of which can be secured to another additional box like case and in an intermediate position between saddle-like contact part and back side of the case of the main unit.

## Patentansprüche

1. Tragbares Ultraschalldiagnosesystem, umfassend:
eine Sonde zum Übertragen und Empfangen eines Ultraschallsignals an einen interessierenden Bereich von demselben;
ein Hauptteil, der Schaltungen in Kommunikation mit der Sonde zum Bilden eines Bildes des interessierenden Bereichs auf der Basis des Ultraschallsignals umfasst; und
eine Anzeige zum Darstellen des gebildeten Bildes;
wobei die Sonde mit den Schaltungen im Hauptteil mittels einer Kabelverbindung verbunden ist;
wobei der Hauptteil ein Gewicht hat, das von einem Arm getragen werden kann; und
wobei der Hauptteil mit einem Griff zum Halten mit einer Hand versehen ist;
**dadurch gekennzeichnet, dass**
der Hauptteil ein Gehäuse in Form eines Tablet-Computers hat, der eine Vorderseite hat, die durch die Anzeige gebildet wird, und eine Rückseite;
wobei der Griff durch ein längliches Element gebildet wird, das am Gehäuse des Hauptteils durch freitragende Arme gesichert sind, welche aus dem Gehäuse in einer Richtung der Rückseite des Gehäuses des Hauptteils vorragen und hinter der Rückseite enden, so dass das längliche Element, das den Griff bildet, sich in einem bestimmten Abstand hinter der Rückseite des Gehäuses des Hauptteils erstreckt und näher an einer der zwei entgegengesetzten peripheren Kanten des Gehäuses des Hauptteils ist, derart dass beim Ergreifen des Griffs mit der Hand das Gehäuse des Hauptteils auf dem Unterarm ruhen kann, wobei das Gewicht des Hauptteils vom Unterarm getragen wird und die Hand nur die Funktion hat, das Gehäuse des Hauptteils in seiner Position zu halten.

2. Tragbares Ultraschallsystem nach Anspruch 1, **dadurch gekennzeichnet, dass** das Hauptteilgehäuse die Form eines flachen Parallelepipeds mit zwei größeren Seiten, die die Vorder- und die Rückseite bilden und die durch kleinere Seiten verbunden sind, welche die Seitenflächen sind.

3. Tragbares Ultraschallsystem nach Anspruch 1 oder 2, **dadurch gekennzeichnet, dass** die Vorder- und Rückseiten des Gehäuses des Hauptteils rechteckig sind.

4. Tragbares Ultraschallsystem nach einem oder mehreren der vorherigen Ansprüche, **dadurch gekennzeichnet, dass** das längliche Element, das den Griff bildet, in einem Abstand von einer peripheren Seitenkante des Gehäuses des Hauptteils vorgesehen ist, welche auf der entgegengesetzten Seite der peripheren Seitenkante des Gehäuses ist, die dem länglichen Element näher ist, wobei der Abstand nicht größer als der durchschnittliche Abstand von der Handfläche bis zur Ellbogenkehle ist.

5. Tragbares Ultraschallsystem nach einem oder mehreren der vorherigen Ansprüche, **dadurch gekennzeichnet, dass** zum Einstellen des Abstandes des länglichen Elementes von der peripheren Seitenkante auf der Seite des Gehäuses, die der peripheren Seitenkante auf der Seite entgegengesetzt ist, der das längliche Element näher ist, die Arme, die das längliche Element tragen, am Gehäuse des Hauptteils und/oder den Enden des elastischen Elementes in einer schwenkbaren Weise und speziell in einer schwenkbaren Weise um eine Achse befestigt sind, die parallel zur Achse des länglichen Elementes liegt.

6. Tragbares Ultraschallsystem nach einem oder mehreren der vorherigen Ansprüche, **dadurch gekennzeichnet, dass** die Arme, die das längliche Element tragen, teleskopisch in ihrer Länge einstellbar sind.

7. Tragbares Ultraschallsystem nach einem oder mehreren der vorherigen Ansprüche, **dadurch gekennzeichnet, dass** das längliche Element mindestens einen oder mehrere Buttons oder Tasten trägt, die mit den Schaltungen des Hauptteils zum Erzeugen und Senden von Befehlen an den Hauptteil oder Aktivieren von Funktionen des Ultraschallsystems verbunden sind.

8. Tragbares Ultraschallsystem nach einem oder mehreren der vorherigen Ansprüche, **dadurch gekennzeichnet, dass** es vorteilhaft ist, dass das längliche Element, das den Griff bildet, parallel zu einer Randkante der Anzeige orientiert ist.

9. Tragbares Ultraschallsystem nach einem oder mehreren der vorherigen Ansprüche, **dadurch gekennzeichnet, dass** das Gehäuse des Hauptteils leicht in einem Hoch- oder in einem Querformat der Anzeige getragen werden kann, das heißt, entsprechend den zwei Orientierungen der Anzeige, die gegenüber der anderen um 90° um eine Rotationsachse gedreht werden, welche senkrecht zur Anzeige ist und durch die Mitte der Anzeigefläche verläuft, wobei das Gehäuse die Form eines rechteckigen Parallelepipeds hat, und wobei in der ersten Querformatausrichtung das längliche Element, das den Griff des Gehäuses des Hauptteils bildet, entlang einer horizontalen Richtung ausgerichtet ist, während in der zweiten Hochformatausrichtung das längliche Element entlang einer vertikalen Richtung ausgerichtet ist, und dadurch, dass für eine sichere Abstützung des Hauptteilgehäuses sowohl in der ersten wie auch in der zweiten Ausrichtung die Länge des Hauptteilgehäuses in der Richtung parallel zur Längsachse des länglichen Elementes kleiner als das Doppelte der durchschnittlichen Entfernung von der Handfläche bis zur Ellbogenkehle ist.

10. Tragbares Ultraschallsystem nach einem oder mehreren der vorherigen Ansprüche, **dadurch gekennzeichnet, dass** an dem länglichen Element manuell aktivierbare Steuermittel vorgesehen sind, wie zum Beispiel Tasten, Buttons, Cursors oder dergleichen und zum weiteren Erleichtern des Tragens des Hauptteils, wobei die Spannung oder die Arbeit der Hand zum festen Arretieren des Gehäuses beim Ergreifen des Griffs reduziert wird und auf diese Weise die Verwendung der Hand zum Drücken der manuell aktivierbaren Steuermittel ermöglicht wird, die auf dem länglichen Element angeordnet sind, kann ein Befestigungselement am Unterarm des Systemnutzers derart befestigt werden, dass die Anzeige für den Systemnutzer während des Gebrauchs sichtbar ist, und hat das Befestigungselement ein Straffungsmittel um den Unterarm zum Befestigen des Gehäuses am Unterarm.

11. Tragbares Ultraschallsystem nach Anspruch 10, **dadurch gekennzeichnet, dass** das Befestigungselement durch mindestens einen Riemen gebildet wird, der an der Rückseite des Gehäuses des Hauptteils befestigt ist, d.h. an der Seite, die zum Unterarm des Benutzers ausgerichtet ist, und wobei dieser Riemen zwei Enden hat, die Mittel zum Aneinanderbefestigen der zwei Enden und zum Straffen des Riemens um den Unterarm des Nutzers tragen.

12. Tragbares Ultraschallsystem nach Anspruch 11, **dadurch gekennzeichnet, dass** die Riemen in einer 2wischenposition zwischen dem länglichen Element und der Seite des Gehäuses des Hauptteils gegenüber derjenigen, die dem länglichen Element näher ist, vorgesehen sind.

13. Tragbares Ultraschallsystem nach Anspruch 11 oder 12, **dadurch gekennzeichnet, dass** der mindestens eine Riemen an der Rückseite des Gehäuses des Hauptteils derart befestigt werden kann, dass er um eine Achse senkrecht zur Rückseite des Gehäuses des Hauptteils drehbar ist, und derart, dass der Hauptteil in eine Ausrichtung aus der ersten und zweiten Ausrichtung der Anzeige gedreht werden kann, wenn der Riemen fest am Unterarm des Benutzers angezogen ist.

14. Tragbares Ultraschallsystem nach einem oder mehreren der vorherigen Ansprüche 10 bis 13, **dadurch gekennzeichnet, dass** das Befestigungselement durch eine Kombination eines Riemens und eines anatomischen sattelartigen Kontaktteils gebildet wird, der an den Unterarm angepasst ist, wobei der sattelartige Kontaktteil an der Rückseite des Gehäuses des Hauptteils um eine Achse senkrecht zur Rückseite drehbar befestigt ist.

15. Tragbares Ultraschallsystem nach Anspruch 14, **dadurch gekennzeichnet, dass** Mittel zum lösbaren Stoppen des sattelartigen Kontaktteils in einer Winkelposition aus den mindestens zwei Winkelpositionen vorgesehen sind, die der ersten und zweiten Ausrichtung der Anzeige entsprechen.

16. Tragbares Ultraschallsystem nach Anspruch 14 oder 15, **dadurch gekennzeichnet, dass** zusätzliche Batteriesätze und/oder zusätzliche Hardware in dem sattelartigen Kontaktteil untergebracht ist.

17. Tragbares Ultraschallsystem nach Anspruch 16, **dadurch gekennzeichnet, dass** der sattelartige Kontaktteil lösbar an der Rückseite des Gehäuses des Hauptteils befestigt ist, zwei oder mehr sattelartige Kontaktteile vorgesehen sind, von denen eins oder mehrere sattelartige Kontaktteile voneinander in ihren Abmessungen abweichen, um so eine unterschiedliche Zahl von Batteriesätzen und/oder einer oder mehreren verschiedenen zusätzlichen Hardwareteilen aufnehmen zu können.

18. Tragbares Ultraschallsystem nach Anspruch 16 oder 17, **dadurch gekennzeichnet, dass** der sattelartige Kontaktteil als eine Einheit aufgebaut ist, die durch mehrere Teile gebildet wird, welche aneinander befestigt werden können, wenn ein zusätzliches kastenartiges Gehäuse benötigt wird, das bereitgestellt wird und Mittel zum Befestigen in einer Zwischenposition zwischen dem sattelartigen Kontaktteil und der Rückseite des Gehäuses des Hauptteils hat, während das zusätzliche zwischengeschaltete kastenartige Gehäuse auf einer Seite, die mit der Rückseite des Gehäuses des Hauptteils verbunden werden muss, dasselbe Befestigungsmittel wie das auf der Seite des sattelartigen Kontaktteils hat, während auf der Seite, die mit dem sattelartigen Kontaktteil verbunden werden muss, es dieselben Befestigungsmittel wie die auf der Rückseite des Gehäuses des Hauptteils hat.

19. Tragbares Ultraschallsystem nach Anspruch 18, **dadurch gekennzeichnet, dass** mehr als ein kastenartiges Gehäuse vorgesehen ist, von denen jedes an einem anderen zusätzlichen kastenartigen Gehäuse und in einer Zwischenposition zwischen dem sattelartigen Kontaktteil und der Rückseite des Gehäuses des Hauptteils befestigt werden kann.

## Revendications

1. Système de diagnostic à ultrasons portable comprenant :
une sonde pour transmettre et recevoir un signal à ultrasons vers et depuis une région d'intérêt ;
une unité principale comprenant des circuits en communication avec ladite sonde pour former une image de la région d'intérêt sur la base d'un signal à ultrasons et ;
un écran pour afficher l'image formée ;
ladite sonde étant connectée auxdits circuits dans l'unité principale au moyen d'une connexion par câble ;
ladite unité principale ayant un poids qui peut être supporté par un bras et ;
l'unité principale étant équipée d'une poignée pouvant être saisie à une main ;
**caractérisé en ce que** :
l'unité principale possède un boîtier sous la forme d'une tablette numérique, ayant une face avant constituée par l'écran et une face arrière ;
ladite poignée étant formée par un élément allongé qui est fixé au boîtier de l'unité principale par des bras en porte à faux dépassant du boîtier en direction de la face arrière du boîtier de l'unité principale et se terminant derrière ladite face arrière de manière à ce que l'élément allongé formant la poignée s'étende à une certaine distance derrière la face arrière du boîtier de l'unité principale et soit plus proche d'un des deux bords périphériques opposés dudit boîtier de l'unité principale de telle manière que lorsqu'une main saisie la poignée, le boîtier de l'unité principale peut reposer sur l'avant-bras, avec le poids de l'unité principale étant supporté par l'avant-bras et la main ayant seulement la fonction de blocage du boîtier de l'unité principale en position.

2. Système à ultrasons portable selon la revendication 1, **caractérisé en ce que** le boîtier de l'unité principale a la forme d'un paralépipède plat ayant deux grands côtés qui forment les faces avant et arrière et qui sont reliées par des côtés plus petits constituant les côtés latéraux.

3. Système à ultrasons portable selon la revendication 1 ou la revendication 2, **caractérisé en ce que** les faces avant et arrière du boîtier de l'unité principale sont rectangulaires.

4. Système à ultrasons portable selon une ou plusieurs des revendications précédentes, **caractérisé en ce que** l'élément allongé formant la poignée est placé à une distance d'un bord latéral périphérique du boîtier de l'unité principale qui se trouve sur le côté opposé du bord périphérique latéral dudit boîtier qui est plus proche dudit élément allongé, ladite distance n'étant pas plus longue que la distance moyenne s'étendant de la paume de la main au creux du coude.

5. Système à ultrasons portable selon une ou plusieurs des revendications précédentes, **caractérisé en ce que**, de manière à régler ladite distance de l'élément allongé depuis le bord périphérique latéral du côté du boîtier opposé au bord périphérique latéral duquel ledit élément allongé est le plus proche, les bras supportant l'élément allongé sont fixés au boîtier de l'unité principale et/ou aux extrémités de l'élément allongé d'une manière rotative et, en particulier, d'une manière rotative autour d'un axe qui est parallèle à l'axe de l'élément allongé.

6. Système à ultrasons portable selon une ou plusieurs des revendications précédentes, **caractérisé en ce que** les bras supportant l'élément allongé sont réglables, de manière télescopique, en longueur.

7. Système à ultrasons portable selon une ou plusieurs des revendications précédentes, **caractérisé en ce que** l'élément allongé comporte au moins un ou plusieurs boutons ou clés de contrôle, qui sont connectés aux circuits de l'unité principale pour générer et envoyer des commandes à ladite unité principale et pour activer ou désactiver des fonctions du système à ultrasons.

8. Système à ultrasons portable selon une ou plusieurs des revendications précédentes, **caractérisé en ce que** l'élément allongé formant la poignée est orienté avantageusement de manière parallèle par rapport à un bord périphérique de l'écran.

9. Système à ultrasons portable selon une ou plusieurs des revendications précédentes, **caractérisé en ce que** le boîtier de l'unité principale peut être supporté facilement soit dans une orientation d'écran dite portrait, soit dite paysage, ceci signifiant, conformément aux deux orientations de l'écran qui sont tournées à 90° l'une par rapport à l'autre autour d'un axe de rotation qui est perpendiculaire à l'écran et traverse le centre de la zone d'affichage ;
le boîtier ayant une forme de parallélépipède rectangle et dans ladite première orientation paysage, l'élément allongé formant la poignée du boîtier de l'unité principale est orienté suivant une direction horizontale, tandis que dans ladite seconde orientation portrait, l'élément allongé est orienté suivant une direction verticale ; et **en ce que** afin d'avoir un maintien sûr du boîtier de l'unité principale à la fois dans lesdites première et seconde orientations, la longueur du boîtier principal dans la direction parallèle à l'axe longitudinal de l'élément allongé est inférieure à deux fois la distance moyenne s'étendant de la paume de la main au creux du coude.

10. Système à ultrasons portable selon une ou plusieurs des revendications précédentes, **caractérisé en ce que** sur l'élément allongé, des moyens de contrôle activables manuellement sont fournis tels que des clés, des boutons, des curseurs ou similaires et afin de faciliter en outre le maintien de l'unité principale, en réduisant la contrainte ou le travail de la main pour bloquer fermement le boîtier en agrippant la poignée et ainsi permettant l'utilisation de ladite main pour presser les moyens de contrôle activables manuellement disposés sur l'élément allongé, un élément de fixation est fourni pour ledit boîtier de l'unité principale, ledit élément de fixation pouvant être monté autour de l'avant-bras de l'utilisateur du système de manière à ce que l'écran soit visible pour l'utilisateur du système au cours de l'utilisation, et ledit élément de fixation ayant de moyens de serrage autour de l'avant-bras pour fixer le boîtier à l'avant-bras.

11. Système à ultrasons portable selon la revendication 10, **caractérisé en ce que** l'élément de fixation est formé par au moins une sangle fixée à la face arrière du boîtier de l'unité principale, c'est-à-dire la face orientée vers l'avant-bras de l'utilisateur, et laquelle sangle possède deux extrémités qui comportent des moyens pour fixer lesdites deux extrémités l'une à l'autre et pour serrer la sangle autour de l'avant-bras de l'utilisateur.

12. Système à ultrasons portable selon la revendication 11, **caractérisé en ce que** les sangles sont placées dans une position intermédiaire entre l'élément allongé et la face du boîtier de l'unité principale opposée à celle qui est la plus proche de l'élément allongé.

13. Système à ultrasons portable selon la revendication 11 ou la revendication 12, **caractérisé en ce que** l'au moins une sangle peut être fixée à l'arrière du boîtier de l'unité principale de manière à pouvoir pivoter autour d'un axe perpendiculaire à ladite face arrière du boîtier de l'unité principale et de manière à ce que l'unité principale puisse pivoter dans l'une desdites première et seconde orientations de l'écran lorsque la sangle est fixée fermement à l'avant-bras de l'utilisateur.

14. Système à ultrasons portable selon une ou plusieurs des revendications précédentes 10 à 13, **caractérisé en ce que** l'élément de fixation est formé par une combinaison d'une sangle et d'une partie de contact anatomique semblable à une selle qui est placée sur l'avant-bras, la partie de contact semblable à une selle étant fixée à la face arrière du boîtier de l'unité principale de manière à pouvoir pivoter autour d'un axe perpendiculaire de ladite face arrière.

15. Système à ultrasons portable selon la revendication 14, **caractérisé en ce que** des moyens sont fournis pour arrêter de manière libérable ladite partie de contact semblable à une selle dans une position angulaire des au moins deux positions angulaires correspondant auxdites première et secondes orientations de l'écran.

16. Système à ultrasons portable selon la revendication 14 ou la revendication 15, **caractérisé en ce que** des blocs de batteries supplémentaires et/ou du matériel supplémentaire sont logés dans la partie de contact anatomique semblable à une selle.

17. Système à ultrasons portable selon la revendication 16, **caractérisé en ce que** ladite partie de contact semblable à une selle est fixée de manière libérable à la face arrière du boîtier de l'unité principale, deux ou plusieurs de parties de contact semblables à une selle sont fournies laquelle ou lesquelles partie de contact semblable à une selle diffèrent les unes des autres par rapport à leurs dimensions afin de pouvoir loger un nombre différent de blocs de batteries et/ou une ou plusieurs unités de matériel supplémentaires différentes.

18. Système à ultrasons portable selon la revendication 16 ou la revendication 17, **caractérisé en ce que** ladite partie de contact semblable à une selle est construite comme une unité formée de plusieurs parties qui peuvent être assemblées les unes aux les autres lorsque nécessaire, un boîtier en forme de boîte supplémentaire, étant fourni, ayant des moyens pour être fixé dans une position intermédiaire entre la partie de contact semblable à une selle et la face arrière du boîtier de l'unité principale, tandis que ledit boîtier en forme de boîte intermédiaire supplémentaire dispose, sur une face à connecter à la face arrière du boîtier de l'unité principale, des mêmes moyens de connexion que ceux situés sur la face de la partie de contact semblable à une selle, tandis que sur la face à connecter à ladite partie de contact semblable à une selle, il dispose des mêmes moyens de connexion que ceux fournis sur la face arrière du boîtier de l'unité principale.

19. Système à ultrasons portable selon la revendication 18, **caractérisé en ce que** plus d'un boîtier en forme de boîte supplémentaires sont fournis, chacun d'entre eux pouvant être fixé à un autre boîtier en forme de boîte supplémentaire et dans une position intermédiaire entre la partie de contact semblable à une selle et la face arrière du boîtier de l'unité principale.
